# EUROPEAN PATENT APPLICATION

(11) **EP 4 620 480 A1**
(43) Date of publication of application: **24.09.2025**
(21) Application number: 23891591.2
(22) Date of filing: 14.11.2023
(51) Int. Cl.: A61K 38/10, A61K 35/48, A61K 45/00, A61K 47/54, A61L 27/00, A61L 27/36, A61L 27/52, A61P 15/00, C07K 7/08, C12N 5/071, C12Q 1/02

(54) **IMPLANTATION FACILITATOR, AND SCREENING SYSTEM THEREFOR**

(30) Priority: 16.11.2022 JP 2022183639
(71) Applicant: Kansai Medical University Educational Corporation, City of Hirakata, Osaka 573-1010 (JP)
(72) Inventor: MATSU-URA, Toru, Hirakata City, Osaka 5731010 (JP); IKEDA, Yoshiki, Kyoto-shi, Kyoto 606-8501 (JP); YOSHIDA, Naoko, Hirakata City, Osaka 5731010 (JP)
(74) Representative: J A Kemp LLP
(86) International application number: PCT/JP2023/041000
(87) International publication number: WO 2024/106444

(57) **Abstract**

The present invention provides a medicament which can improve the implantation rate of an embryo and can be used in combination with an existing medicament used in the treatment of infertility, a screening method by which such a medicament can be reliably evaluated, etc. More specifically, provided is an integrin activator comprising: (1) a peptide consisting of KFEEERMRCKWMT; (2) a peptide consisting of KFEEERSRCKWMT; (3) in (1) or (2), a peptide consisting of an amino acid sequence having one to three amino acids deleted, substituted and/or added thereto; or (4) in any one of (1) to (3), a peptide having a membrane-permeable molecule bound thereto. Provided are: an artificial uterus comprising a hydrogel which contains endometrial stromal cells and has extracellular matrix as a main component, and a uterine epithelial organoid which is arranged so as to be surrounded by said hydrogel; and a screening method using an implantation reproduction experimental system using a fertilized egg (e.g., an embryo which has been developed to a blastocyst to be implanted).

## Description

### FIELD

The present invention relates to: agents for activating integrins, containing a specific peptide; an artificial uterus including a hydrogel that contains endometrial stromal cells and is mainly composed of extracellular matrix and an endometrial epithelial organoid arranged so as to be surrounded by the hydrogel; a screening method for an agent for treating infertility, performed using the artificial uterus; and so forth.

### BACKGROUND

In developed countries, the number of patients undergoing fertility treatments is increasing under social conditions that have led to the trend toward late marriage. Also, in our country Japan, where measures against the declining birthrate are urgently needed, the number of fertility treatments has exceeded 300,000 owing to the trend toward late marriage. Fertility treatments involve three stages of treatments: hormonal treatments to improve the condition of the uterus; artificial insemination performed by artificially injecting sperm and an egg into the uterus; and treatments using assisted reproductive technologies such as in vitro fertilization and microfertilization. In particular, emphasis is placed on fertility treatments focusing on gametes, such as artificial insemination and microfertilization. Existing fertility treatments can dramatically increase the chances of sperm and egg meeting. However, implantation, an event that occurs subsequently, is merely left to nature.

Although existing fertility treatments let implantation take its own course as described above, aging not only deteriorates the quality of gametes but also markedly decreases the efficiency of implantation of fertilized eggs in the uterus. Accordingly, the implantation rate of artificially inseminated embryos decreases with age, and is 35% in patients in their early thirties, 25% in patients in their late thirties, and 10% or less in patients in their forties or older, for example.

Although treatment methods that directly act on implantation have been investigated, methods that effectively improve the implantation have not yet been found. Under such circumstances, there is demand for development of medicaments that can improve the embryo implantation rate and also can be used in combination with medicaments used in conventional fertility treatments (Patent Document 1). At the same time, there is also demand for establishment of a screening system that enables reliable evaluation of such medicaments.

### [CITATION LIST]

### [PATENT LITERATURE]

[PTL 1] International Patent Publication No. WO2019/101368

### SUMMARY

### [TECHNICAL PROBLEM]

With the foregoing in mind, it is an object of the present invention to provide a medicament that can improve the embryo implantation rate and also can be used in combination with medicaments used in existing fertility treatments, a screening method that enables reliable evaluation of such medicaments, and so forth.

### [SOLUTION TO PROBLEM]

The inventors of the present invention investigated existing fertility treatments, and focused their attention to the fact that, although these treatments can dramatically increase the chances of sperm and egg meeting, implantation, which occurs subsequently, is merely left to nature. Then, in an attempt to find substances that affect the implantation rate through screening, the inventors first came up with the idea of establishing a screening system by simplifying the complex mechanism of an actual event implantation occurring in vivo, in which various mechanisms act from both the endometrium and an embryo to cause the attachment of the embryo and subsequent invasion of the embryo into the endometrial stromal layer. The inventors expected that establishing such a screening system enables quick and reliable evaluation of a large number of samples, leading to acquisition of substances that can improve the implantation rate. In light of the foregoing, the inventors came up with the idea of controlling the environment in which an endometrial epithelial organoid is to be placed and the polarity of the endometrial epithelial organoid, thereby allowing natural implantation to take place merely by bringing an embryo (blastocyst) into contact with the endometrial epithelial organoid. The inventors conducted in-depth studies based on these ideas and succeeded in establishing the screening method and the like of the present invention.

The inventors of the present invention conducted further studies to find out what kinds of substances should be targeted in an attempt to improve the embryo implantation rate. In the course of the studies, the inventors focused on attachment between endometrial epithelial cells and trophoblastic cells of embryos, and directed their attention to integrins involved in the attachment. The inventors then came up with the idea that it might be possible to improve the embryo implantation rate by using substances that activate integrins, which attracted their attention.

In order to make the above idea concrete, the inventors used the completed screening method of the present invention to examine whether the substances that activate integrins can actually improve the embryo implantation rate. To this end, the inventors predicted the structures of substances that might change the activity of integrins based on the three-dimensional structures and the like of the integrins, and evaluated over 60,000 substances. Based on these findings, the inventors conducted further in-depth studies, which led to completion of the present invention.

Specifically, the present invention provides the following.
[1] An agent for activating integrins, containing:
   (1) a peptide consisting of KFEEERMRCKWMT;
   (2) a peptide consisting of KFEEERSRCKWMT;
   (3) a peptide consisting of the amino acid sequence specified in (1) or (2) with deletion, substitution, and/or addition of one to three amino acids; or
   (4) the peptide of any one of (1) to (3) with a membrane-permeable molecule bound thereto.
[2] The agent according to [1], wherein the membrane-permeable molecule is myristic acid.
[3] The agent according to [1] or [2] for promoting embryo implantation.
[4] An agent for treating infertility, containing:
   the agent according to any one of [1] to [3]; and
   a hormonal agent.
[5] An artificial uterus including:
   a hydrogel containing endometrial stromal cells and mainly composed of extracellular matrix; and
   an endometrial epithelial organoid arranged so as to be surrounded by the hydrogel.
[6] The artificial uterus according to [5], wherein the hydrogel arranged so as to surround the endometrial epithelial organoid has an opening open to the top thereof.
[7] The artificial uterus according to [5] or [6], wherein the extracellular matrix contains laminin and/or a fragment thereof.
[8] The artificial uterus according to any one of [5] to [7], wherein an outer side of the endometrial epithelial organoid is an apical side.
[9] A kit for producing an artificial uterus, including:
   a device including a support with an objective surface and at least one protrusion protruding from the objective surface; and
   a solution mainly composed of extracellular matrix.
[10] A method for producing an artificial uterus, including the steps of:
   (1) filling a culture vessel with a solution containing endometrial stromal cells and mainly composed of extracellular matrix;
   (2) pressing a device including a support with an objective surface and at least one protrusion protruding from the objective surface against the solution filling the culture vessel; and
   (3) after the solution filling the culture vessel has turned into a gel, removing the device pressed against the solution.
[11] A screening method for an agent for treating infertility, including the steps of:
   (1) bringing a fertilized egg into contact with the artificial uterus according to any one of [5] to [8] in the presence or absence of a test substance;
   (2) measuring an implantation rate of the fertilized egg in the endometrial epithelial organoid; and
   (3) when the implantation rate is higher in the presence of the test substance than in the absence of the test substance in the step (2), selecting the test substance as a candidate substance for a therapeutic or prophylactic medicament for infertility.
[12] A method for promoting embryo implantation in a subject, including administering the agent according to [1] or [2].
[13] A method for treating infertility in a subject, including administering a therapeutically effective amount of the agent according to [1] or [2].
[14] The method according to [13], wherein the agent further contains a hormonal agent.
   [I] An agent for promoting embryo implantation or treating infertility, containing:
      (1) a peptide consisting of KFEEERMRCKWMT;
      (2) a peptide consisting of KFEEERSRCKWMT;
      (3) a peptide consisting of the amino acid sequence specified in (1) or (2) with deletion, substitution, and/or addition of one to three amino acids; or
      (4) the peptide of any one of (1) to (3) with a membrane-permeable molecule bound thereto.
   [II] The agent according to [I], wherein the peptide with the membrane-permeable molecule bound thereto is a myristoylated peptide.
   [III] The agent according to [I] or [II], further containing a hormonal agent.
   [IV] An agent for activating integrins, containing:
      (1) a peptide consisting of KFEEERMRCKWMT;
      (2) a peptide consisting of KFEEERSRCKWMT;
      (3) a peptide consisting of the amino acid sequence specified in (1) or (2) with deletion, substitution, and/or addition of one to three amino acids; or
      (4) the peptide of any one of (1) to (3) with a membrane-permeable molecule bound thereto.
   [V] An artificial uterus including:
      a hydrogel containing endometrial stromal cells and mainly composed of extracellular matrix; and
      an endometrial epithelial organoid arranged so as to be surrounded by the hydrogel,
      wherein the hydrogel arranged so as to surround the endometrial epithelial organoid has an opening open to the top thereof.
   [VI] The artificial uterus according to [V], wherein the extracellular matrix contains laminin and/or a fragment thereof.
   [VII] The artificial uterus according to [V], wherein an outer side of the endometrial epithelial organoid is an apical side.
   [VIII] A kit for producing an artificial uterus, including:
      a device including a support with an objective surface and at least one protrusion protruding from the objective surface; and
      a solution mainly composed of extracellular matrix.
   [IX] A method for producing an artificial uterus, including the steps of:
      (1) filling a culture vessel with a solution containing endometrial stromal cells and mainly composed of extracellular matrix;
      (2) pressing a device including a support with an objective surface and at least one protrusion protruding from the objective surface against the solution filling the culture vessel; and
      (3) after the solution filling the culture vessel has turned into a gel, removing the device pressed against the solution.
   [X] A screening method for an agent for treating infertility, including the steps of:
      (1) bringing a fertilized egg into contact with the artificial uterus according to [V] in the presence or absence of a test substance;
      (2) measuring an implantation rate of the fertilized egg in the endometrial epithelial organoid; and
      (3) when the implantation rate is higher in the presence of the test substance than in the absence of the test substance in the step (2), selecting the test substance as a candidate substance for a therapeutic or prophylactic medicament for infertility.
   [XI] A peptide as set forth in any of the following (1) to (4) for use in promotion of embryo implantation or treatment of infertility:
      (1) a peptide consisting of KFEEERMRCKWMT;
      (2) a peptide consisting of KFEEERSRCKWMT;
      (3) a peptide consisting of the amino acid sequence specified in (1) or (2) with deletion, substitution, and/or addition of one to three amino acids; or
      (4) the peptide of any one of (1) to (3) with a membrane-permeable molecule bound thereto.
   [XII] A peptide as set forth in any of the following (1) to (4) for use in integrin activation:
      (1) a peptide consisting of KFEEERMRCKWMT;
      (2) a peptide consisting of KFEEERSRCKWMT;
      (3) a peptide consisting of the amino acid sequence specified in (1) or (2) with deletion, substitution, and/or addition of one to three amino acids; or
      (4) the peptide of any one of (1) to (3) with a membrane-permeable molecule bound thereto.
   [XIII] The peptide according to [XI] or [XII], wherein the peptide with the membrane-permeable molecule bound thereto is a myristoylated peptide.
   [XIV] Use of a peptide as set forth in any of the following (1) to (4) for production of an agent for promoting embryo implantation or treating infertility:
      (1) a peptide consisting of KFEEERMRCKWMT;
      (2) a peptide consisting of KFEEERSRCKWMT;
      (3) a peptide consisting of the amino acid sequence specified in (1) or (2) with deletion, substitution, and/or addition of one to three amino acids; or
      (4) the peptide of any one of (1) to (3) with a membrane-permeable molecule bound thereto.
   [XV] Use of a peptide as set forth in any of the following (1) to (4) for production of an agent for activating integrins:
      (1) a peptide consisting of KFEEERMRCKWMT;
      (2) a peptide consisting of KFEEERSRCKWMT;
      (3) a peptide consisting of the amino acid sequence specified in (1) or (2) with deletion, substitution, and/or addition of one to three amino acids; or
      (4) the peptide of any one of (1) to (3) with a membrane-permeable molecule bound thereto.
   [XVI] The use according to [XIV] or [XV], wherein the peptide with the membrane-permeable molecule bound thereto is a myristoylated peptide.

### [ADVANTAGEOUS EFFECTS OF INVENTION]

The present invention can improve the embryo implantation rate after natural conception, artificial insemination, and the like by activating integrins. In addition, since the agent according to the present invention can be used in combination with medicaments used in conventional fertility treatments, it can serve as one of solutions to the declining birthrate and also contributes to reduction of cost for fertility treatments, as well as reduction of increasing subsidies and the like for such treatments. Moreover, the present invention enables quick and reliable evaluation of substances that can improve the embryo implantation rate and thus is very useful for obtaining such substances.

### BRIEF DESCRIPTION OF DRAWINGS

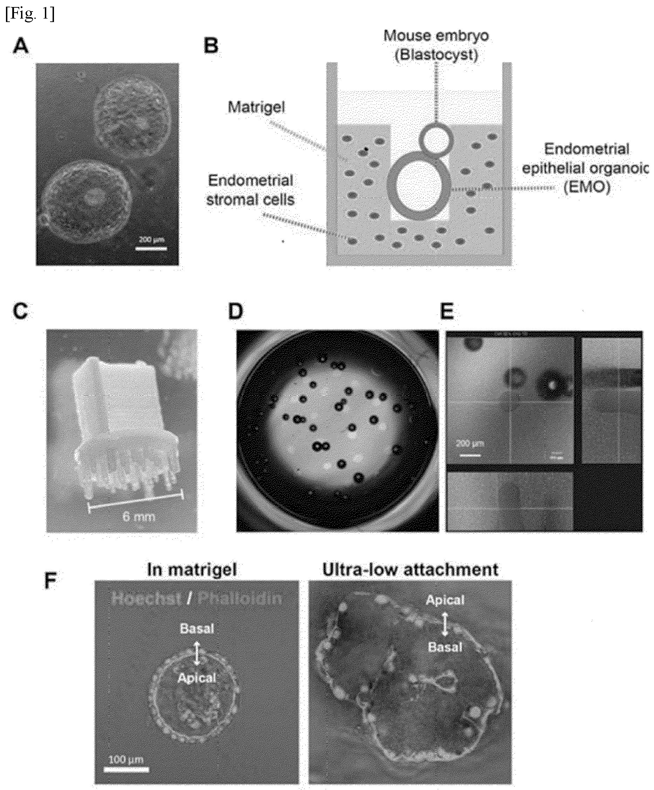

[FIG. 1] FIG. 1 (FIGs. 1A to 1F) illustrates an overview of the artificial uterus (in vitro implantation system) according to the present invention. FIG. 1A shows that a uterine organoid is a spherical cell mass composed of a single columnar epithelial cell layer and present in Matrigel. FIG. 1B shows the artificial uterus according to the present invention, in which an endometrial epithelial organoid and a blastocyst from which the zona pellucida has been detached are placed in a hole formed in a hydrogel containing endometrial stromal cells and mainly composed of extracellular matrix (Matrigel^{™}). FIGs. 1C to 1E illustrate production of a structure with holes having a diameter of 200 µm (FIGs. 1D and 1E) by suspending stromal cells in Matrigel, placing the resulting suspension in a glass-bottom dish, and solidifying the suspension with a die produced using a 3D printer (FIG. 1C) placed thereon. The right panel in FIG. 1F shows that, in a uterine organoid cultured in Matrigel, nuclei and actin filaments are oriented from the outside toward the inside of the sphere. The right panel in FIG. 1F shows that, in a uterine organoid cultured in an ultra-low attachment culture vessel (ultra-low attachment dish), nuclei and actin filaments are oriented from the inside toward the outside of the sphere.

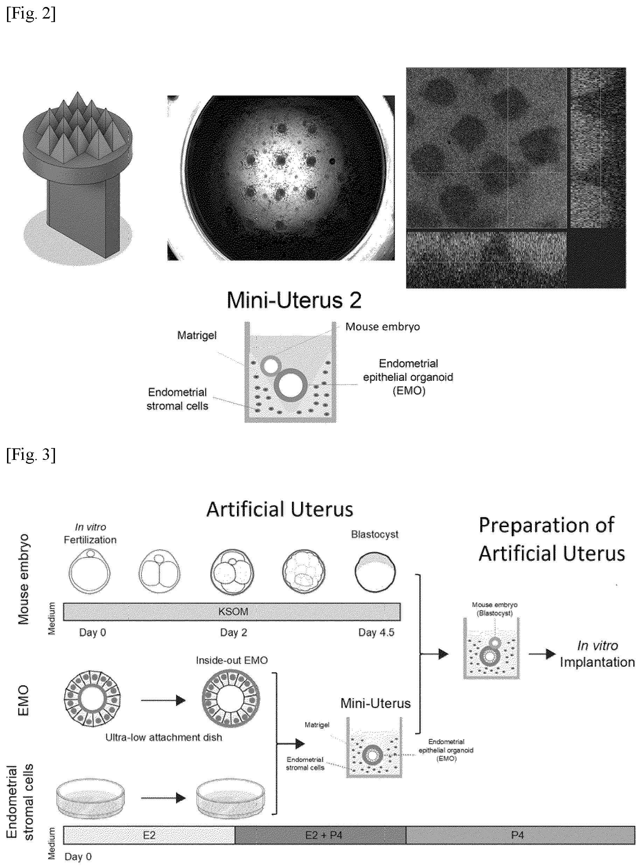

FIG. 2 shows one embodiment of the artificial uterus and the kit for producing the artificial uterus according to the present invention.
FIG. 3 shows the artificial uterus (in vitro implantation system) according to the present invention and an overview of its preparation.

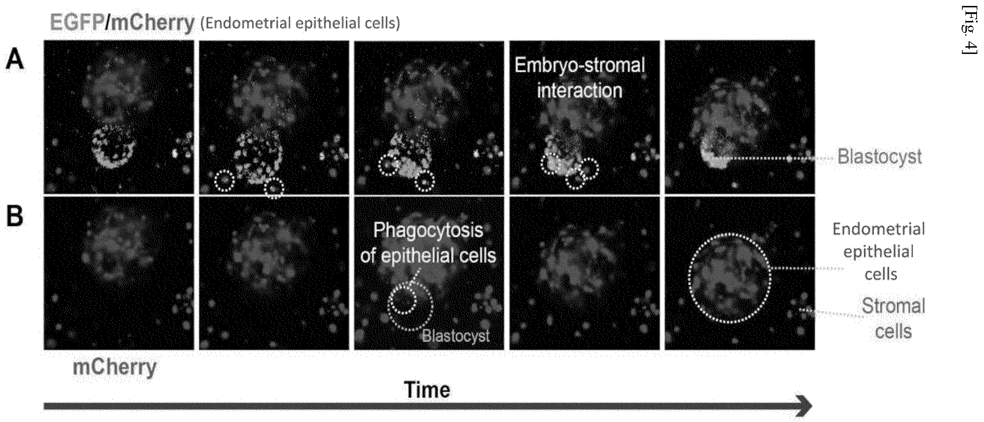

FIG. 4 (FIGs. 4A and 4B) shows the results of observing the in vitro implantation (IVIM) system under a confocal microscope. FIG. 4A shows that a blastocyst, which was first in contact with a uterine organoid, gradually penetrated into the uterine organoid while causing interactions with surrounding stromal cells. FIG. 4B shows that mCherry fluorescence was observed in the blastocyst, suggesting that epithelial cells were removed through phagocytosis to allow penetration.

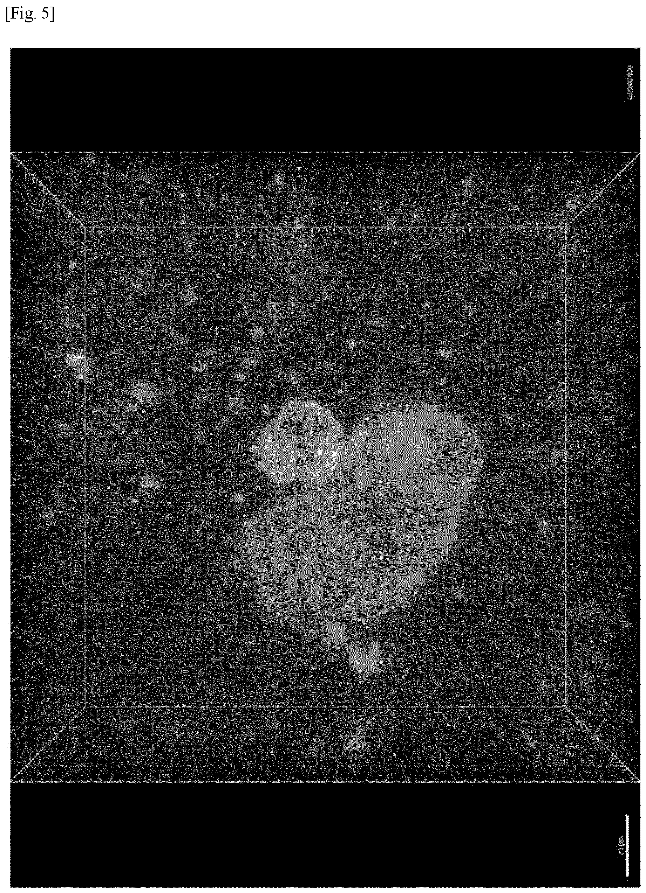

FIG. 5 shows the results regarding the in vitro implantation (IVIM) system. FIG. 5 shows that the results observed in FIG. 4A and 4B were also observed in a blastocyst and a uterine organoid/stromal cells stained with Cell Explorer^{™} Live Cell Tracking Kit Green Fluorescence and Red Fluorescence.

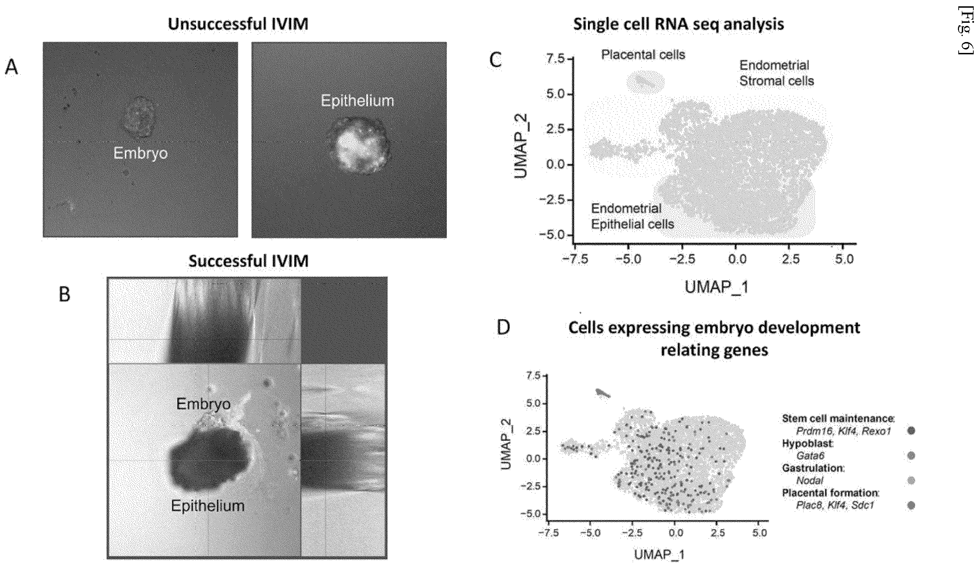

FIG. 6 (FIGs. 6A to 6D) shows the results regarding fusing endometrial epithelium and embryo after IVIM. FIG. 6A shows that, when no implantation-like reaction occurred in an in vitro implantation experiment, an embryo (the left panel in FIG. 6A) and a uterine organoid (the right panel in FIG. 6A) removed from the experimental system remain separated from each other. FIG. 6B shows that, when an implantation-like reaction occurred, the embryo removed from the experimental system was fused with the uterine organoid so as not to separate from each other. FIG. 6C shows the results of single-cell RNA expression analysis performed after placing blastocysts in the in vitro implantation experimental system, removing all cells from the system 72 hours later, and treating the cells with trypsin to disassociate them into single cells. Based on the gene expression patterns, the cells were broadly classified into three groups, namely, placental-like cells, endometrial epithelial cells, and endometrial stromal cells. In addition, cells expressing the following various marker genes were scattered: genes involved in stem cell maintenance, such as Prdm16, Klf4, and Rexo1; a gene involved in endoderm formation, such as Gata6; a gene involved in gastrulation, such as Nodal; and genes involved in placentation, such as Plac8, Klf4, and Sdc1. This suggests that embryo development had progressed beyond the blastocyst stage (FIG. 6D).

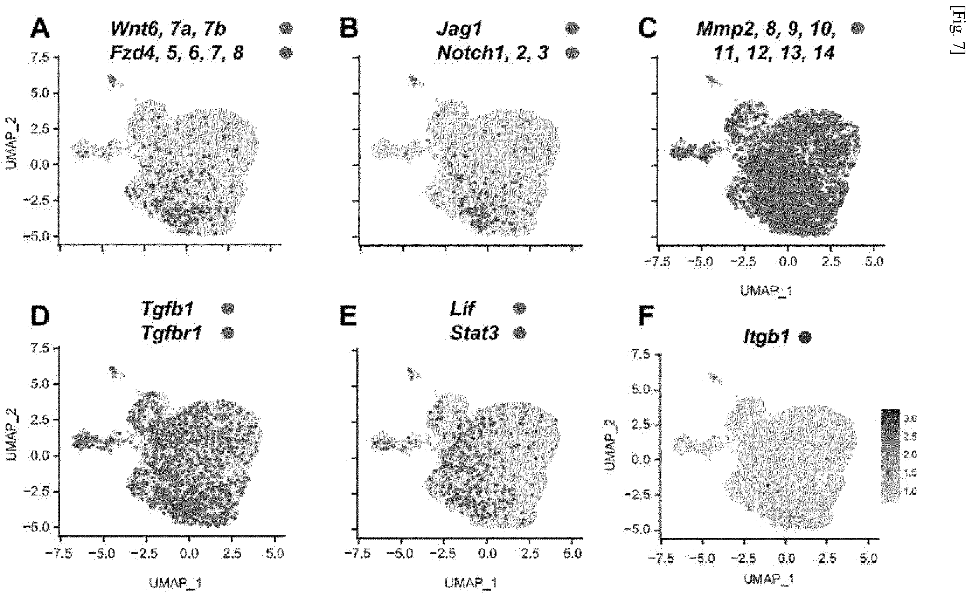

FIG. 7 (FIGs. 7A to 7F) shows the results of single-cell RNA expression analysis performed to confirm the expression of genes known to be involved in implantation. The results show cells expressing implantation-related genes such as WNT signals (FIG. 7A), Notch signals (FIG. 7B), metalloproteases (FIG. 7C), TGFβ (FIG. 7D), LIF (FIG. 7E), and an integrin (FIG. 7F).

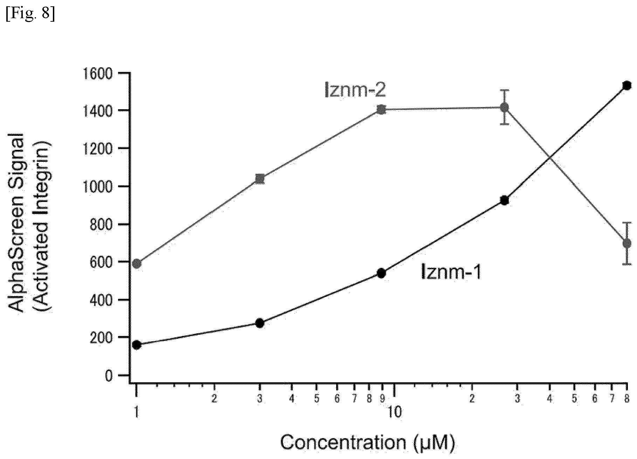

FIG. 8 shows the results of measuring the integrin activation ability of two peptides (Iznm-1 and Iznm-2) selected from over 60,000 types of peptides. The measurement confirmed that Iznm-1 can exhibit approximately 5-fold activation effect and Iznm-2 can exhibit approximately 14-fold activation effect at a concentration of 8 µM.

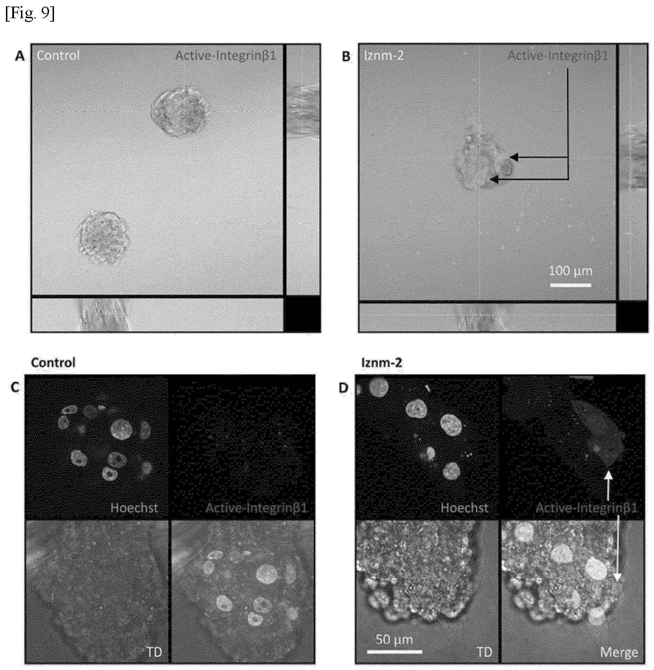

FIG. 9 (FIGs. 9A to 9D) shows the results confirming that the peptide medicament Iznm-2 causes integrin activation (Active-Integrin β1) in embryos. The results show that cells more strongly fluorescently stained as compared to cells in a control group (FIGs. 9A and 9C) were detected in an experimental group (in particular, portions indicated with arrows) (FIGs. 9B and 9D).

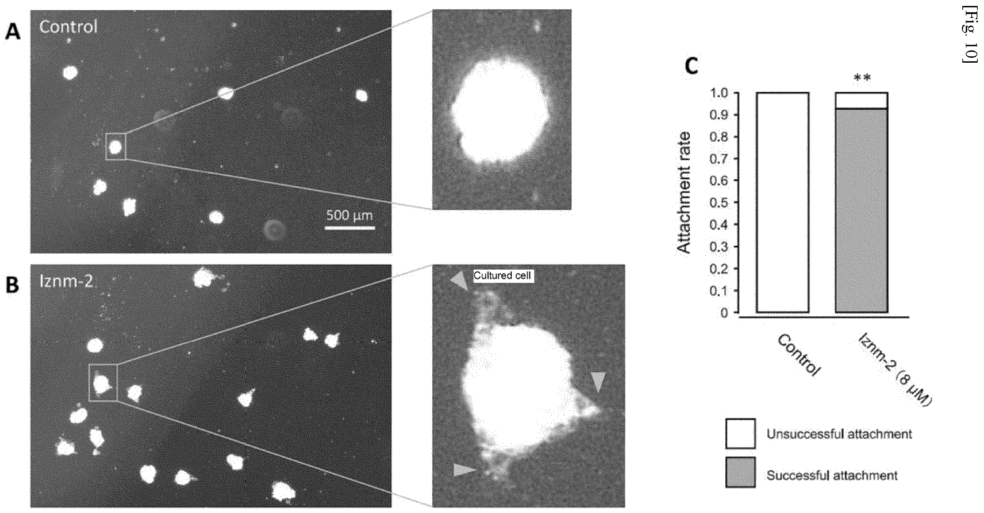

FIG. 10 (FIGs. 10A to 10C) shows the results of examining whether Iznm-2 promotes attachment of embryos (mouse blastocysts) to a glass surface. Embryos in a control group did not attach to the glass surface, whereas, in the Iznm-2-added experimental group, the attachment of embryos to the glass surface was observed (FIGs. 10A and 10B). The rate of attachment was 0/13 in the control group and 13/14 in the Iznm-2-added group, and a significant difference was detected between the two groups (p < 0.01, chi-square test, FIG. 10C).

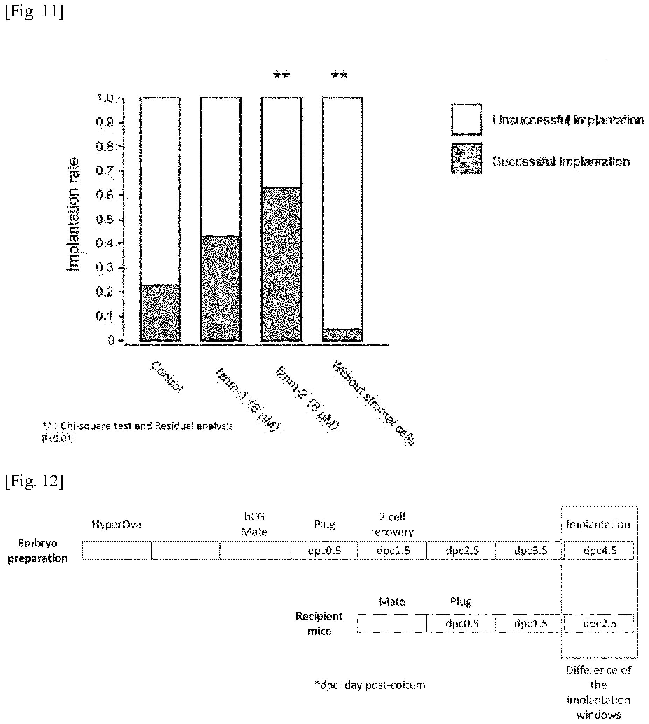

FIG. 11 shows the results regarding the effects of peptide medicaments (Iznm-1 and Iznm-2) in the in vitro implantation system (the rate at which an implantation reaction was observed in the in vitro implantation system).
FIG. 12 illustrates an overview of a test to examine the effects of Iznm-2 on implantation and development of transferred embryos. The state of implantation window shift was reproduced by causing a mismatch between the number of days post-coitum in embryos to be transferred and the number of days post-coitum in recipient mice.

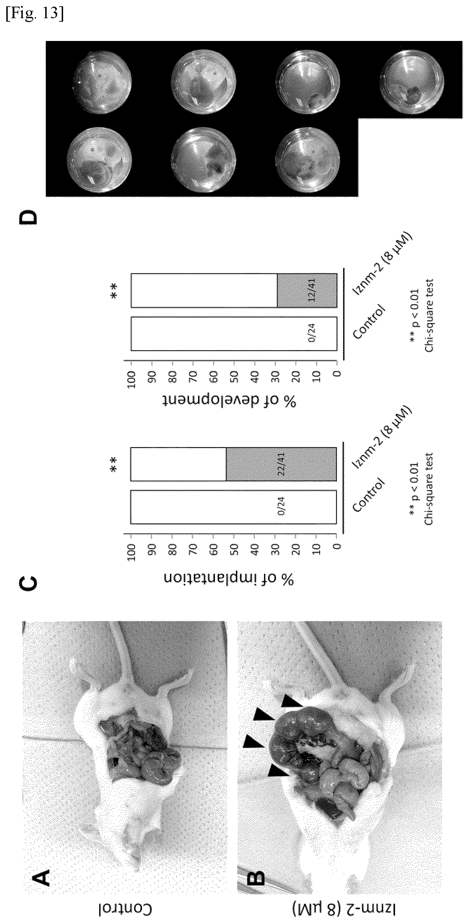

FIG. 13 (FIGs. 13A to 13D) shows the results regarding the effects of Iznm-2 on implantation and development of a transferred embryo. As compared to a control shown in FIG. 13A, a larger number of implanted and developed embryos at the stage of E15.5 were observed in an experimental group shown in FIG. 13B in which 8 µM Iznm-2 had been added at the time of embryo transfer (FIGs. 13A to 13C). In the Iznm-2 group, about half of the implanted embryos developed, whereas the rest did not develop at all (FIGs. 13C and 13D).

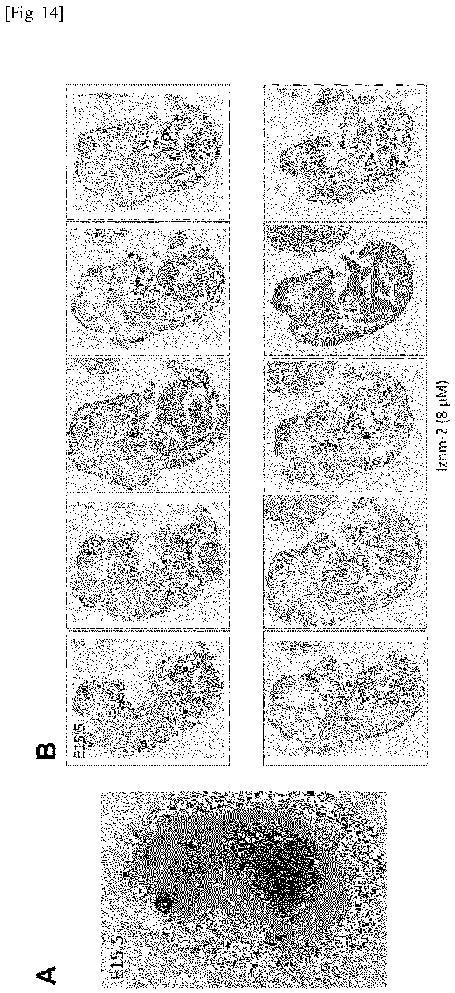

FIG. 14 (FIGs. 14A to 14D) shows the results regarding the effects of Iznm-2 on implantation and development of transferred embryos. FIG. 14A shows the morphology of embryos at E15.5, and FIG. 14B shows HE-stained images of FFPE sections of the embryos also at E15.5.

### DESCRIPTION OF EMBODIMENTS

### 1. Artificial Uterus

The present invention provides an artificial uterus including: a hydrogel containing endometrial stromal cells and mainly composed of extracellular matrix; and an endometrial epithelial organoid arranged so as to be surrounded by the hydrogel.

As used herein, the term "endometrial stromal cells" refers to cells constituting supporting tissue for endometrial epithelial cells and contained in the endometrial stromal layer. The endometrial stromal cells used in the present invention may be: primary endometrial stromal cells isolated from the endometrial tissue; endometrial stromal cells of an established cell line; or endometrial stromal cells induced from (artificial) pluripotent stem cells such as ES cells, nt ES cells, iPS cells, mGS cells, EG cells, or Muse cells.

In the present invention, the density (cells/cm³) of the endometrial stromal cells present in the hydrogel is not limited to a particular density as long as fertilized egg (blastocyst) implantation into the endometrial epithelial organoid to be described below can occur. For example, the density is in the range of 1 × 10⁶ cells/cm³ to 1 × 10⁷ cells/cm³ and preferably in the range of 2 × 10⁶ cells/cm³ to 3 ×10⁶ cells/cm³.

As used herein, the term "endometrial epithelial cells" refers to epithelial cells present in the uterine mucosa (endometrium) and contained in the endometrial epithelial layer. The endometrial epithelial cells used in the present invention may be: primary endometrial epithelial cells isolated from endometrial tissue; an established endometrial epithelial cell line; or endometrial epithelial cells induced from (artificial) pluripotent stem cells such as ES cells, nt ES cells, iPS cells, mGS cells, EG cells, or Muse cells.

As used herein, the term "pluripotent stem cells" refers to stem cells that can differentiate into tissue or cells having various different forms or functions in the body, and that can also differentiate into cells of any lineages of the three germ layers (endoderm, mesoderm and ectoderm). Examples of pluripotent stem cells to be used for the present invention include induced pluripotent stem cells (iPS cells), embryonic stem cells (ES cells), embryonic stem cells from cloned embryos obtained by nuclear transfer (nuclear transfer Embryonic stem cells; ntES cells), multipotent germline stem cells (mGS cells) and embryonic germ cells (EG cells), of which iPS cells (and more preferably human iPS cells) are preferred. When the pluripotent stem cells are ES cells or arbitrary cells derived from human embryos, the cells may be obtained by destruction of the embryo or they may be obtained without destruction of the embryo, but preferably they are cells obtained without destruction of the embryo.

ES cells are stem cells having pluripotency and auto-replicating proliferation potency, established from the inner cell mass of an (early) embryo (such as the blastocyst) of a mammal such as a human or mouse. ES cells were discovered in mice in 1981 (M.J. Evans and M.H. Kaufman (1981), Nature 292:154-156), and ES cell lines were later established in primates including humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cells can be established by extracting the inner cell mass from the blastocyst of a fertilized egg of a target animal and culturing the inner cell mass on a fibroblast feeder. Alternatively, ES cells can be established using a single embryo blastomere alone during the cleavage stage before the blastocyst stage (Chung Y. et al. (2008), Cell Stem Cell 2: 113-117), or they can be established using a developmentally arrested embryo (Zhang X. et al. (2006), Stem Cells 24: 2669-2676.).

ntES cells are ES cells derived from a clone embryo prepared by nuclear transfer, and they have approximately the same properties as fertilized egg-derived ES cells (Wakayama T. et al. (2001), Science, 292:740-743; S. Wakayama et al. (2005), Biol. Reprod., 72:932-936; Byrne J. et al. (2007), Nature, 450:497-502). In other words, ntES (nuclear transfer ES) cells are ES cells established from the inner cell mass of a cloned embryo-derived blastocyst obtained by exchanging an unfertilized egg nucleus with a somatic cell nucleus. Combinations of nuclear transfer (Cibelli J.B. et al. (1998), Nature Biotechnol., 16:642-646) and ES cell preparation techniques (described above) are used to prepare ntES cells (Wakayama, S. (2008), Jikken Igaku, Vol.26, No.5 (special edition), pp.47-52). For nuclear transfer, a somatic cell nucleus may be implanted into a mammalian enucleated unfertilized egg and cultured for several hours for reprogramming.

The ES cell line used for the present invention may be mouse ES cells, and for example, the different mouse ES cell lines established by the inGenious Targeting Laboratory, Riken (Riken Research Institute), etc., may be used, or for human ES cell lines, the different human ES cell lines established by the University of Wisconsin, NIH, Riken, Kyoto University, the National Center for Child Health and Development, Cellartis, etc., for example, may be used. Specific examples of human ES cell lines include CHB-1 to CHB-12 lines, RUES1 line, RUES2 line and HUES1 to HUES28 lines distributed by ESI Bio Co., H1 and H9 lines distributed by WiCell Research, KhES-1, KhES-2, KhES-3, KhES-4, KhES-5, SSES1, SSES2 and SSES3 distributed by Riken, and so on.

iPS cells are cells obtained by reprogramming of mammalian somatic cells or undifferentiated stem cells by introduction of specific factors (nuclear reprogramming factors). A large number of iPS cells currently exist, among which there may be used iPSCs established by introduction of the 4 factors Oct3/4·Sox2·Klf4·c-Myc into mouse fibroblasts by Yamanaka et al. (Takahashi K, Yamanaka S., Cell, (2006) 126: 663-676), iPSCs derived from human cells established by introduction of the same 4 factors into human fibroblasts (Takahashi K, Yamanaka S., et al. Cell, (2007) 131:861-872.), Nanog-iPSCs established by selecting of Nanog expression markers after introduction of the 4 factors (Okita, K., Ichisaka, T. and Yamanaka, S. (2007). Nature 448, 313-317.), iPSCs produced by methods without c-Myc (Nakagawa M, Yamanaka S., et al. Nature Biotechnology, (2008) 26, 101-106), iPSCs established by introduction of 6 factors by a virus-free method (Okita K et al. Nat. Methods 2011 May; 8(5):409-12, Okita K et al. Stem Cells. 31(3):458-66.), and so on. The induced pluripotent stem cells established by introduction of the 4 factors OCT3/4·SOX2·NANOG·LIN28, created by Thomson et al. (Yu J., Thomson JA. et al., Science (2007) 318: 1917-1920), the induced pluripotent stem cells created by Daley et al. (Park IH, Daley GQ. et al., Nature (2007) 451:141-146) and the induced pluripotent stem cells created by Sakurata et al. (Japanese Unexamined Patent Publication No. 2008-307007), etc., may also be used.

In addition, any induced pluripotent stem cells publicly known in the field as described in published journal (for example, Shi Y., Ding S., et al., Cell Stem Cell, (2008) Vol. 3, Issue 5, 568-574, Kim JB., Scholer HR., et al., Nature, (2008) 454, 646-650; Huangfu D., Melton, DA., et al., Nature Biotechnology, (2008) 26, No 7, 795-797), or patent publications (for example, Japanese Unexamined Patent Publication No. 2008-307007, Japanese Unexamined Patent Publication No. 2008-283972, US2008-2336610, US2009-047263, WO2007-069666, WO2008-118220, WO2008-124133, WO2008-151058, WO2009-006930, WO2009-006997 and WO2009-007852), may also be used.

Induced pluripotent stem cell lines that are iPSC lines established by NIH, Riken, Kyoto University, etc., for example, may be used as well. Examples include human iPSC lines such as HiPS-RIKEN-1A line, HiPS-RIKEN-2A line, HiPS-RIKEN-12A line, Nips-B2 line, etc., by Riken, 253G1 line, 253G4 line, 1201C1 line, 1205D1 line, 1210B2 line, 1383D2 line, 1383D6 line, 201B7 line, 409B2 line, 454E2 line, 606A1 line, 610B1 line, 648A1 line, 1231A3 line, FfI-01s04 line, etc., by Kyoto University, with the 1231A3 line being preferred.

mGS cells are pluripotent stem cells derived from the testis and are the source cells for spermatogenesis. Like ES cells, these cells can be induced to differentiate into cells of various lineages, and have properties such as the ability to produce chimeric mice when transplanted into mouse blastocysts (Kanatsu-Shinohara M. et al. (2003) Biol. Reprod., 69:612-616; Shinohara K. et al. (2004), Cell, 119:1001-1012). These cells are capable of auto-replication in a culture medium containing glial cell line-derived neurotrophic factor (GDNF), and by repeated passage under the same culture conditions as those for ES cells, germline stem cells can be obtained (Takebayashi, M. et al. (2008), Jikken Igaku, Vol. 26, No. 5 (special edition), pp. 41-46, Yodosha Co., Ltd. (Tokyo, Japan)).

EG cells are established from the primordial germ cells in the embryonic period and have pluripotency similar to that of ES cells. They can be established by culturing primordial germ cells in the presence of substances such as LIF, bFGF, and stem cell factor (Matsui Y. et al. (1992), Cell, 70:841-847; J. L. Resnick et al. (1992), Nature, 359:550-551).

Pluripotent stem cells in which a gene on a chromosome has been modified using a known genetic engineering technique can also be used in the present invention. The pluripotent stem cells may be adapted such that a marker gene (e.g., a fluorescent protein such as EGFP or GFP) has been knocked in-frame into a gene encoding a differentiation marker using a known method, thereby making it possible to distinguish whether the cells have reached a corresponding differentiation stage with the expression of the marker gene as an indicator.

The source species of the cells (e.g., pluripotent stem cells, endometrial stromal cells, and endometrial epithelial cells) to be used in the present invention is not limited to a particular species, and the cells may be derived from: rodents such as rats, mice, hamsters, and guinea pigs; lagomorphs such as rabbits; ungulates such as pigs, cows, goats, and sheep; dogs; felines such as cats; and primates such as humans, monkeys, rhesus monkeys, marmosets, orangutans, and chimpanzees. Preferable source species are primates. The source species of the cells may be an individual that is the same as or different from an individual from which fertilized eggs (blastocysts or the like) to be applied to the artificial uterus of the present invention is collected. Endometrial cells and fertilized eggs (blastocysts or the like) need not necessarily be derived from the same source species. Also, the source species of cells constituting endometrium-like tissue need not necessarily be the same as the source species of fertilized eggs (blastocysts or the like).

Endometrial stromal cells can be prepared by a method known per se. Specifically, for example, endometrial stromal cells may be obtained by preparing mesenchymal stem cells and then inducing the mesenchymal stem cells to differentiate into endometrial stromal cells based on the description in the Journal of Japan Society for Reproductive Medicine, Vol. 57, No. 4, page 401, October 1, 2012.

The mesenchymal stem cells may be primary cells isolated directly from biological tissue containing them, mesenchymal stem cells of an established cell line, or mesenchymal stem cells obtained by inducing differentiation from ES cells or induced pluripotent stem cells. Such mesenchymal stem cells may be in the cryopreserved state. The term "directly" in this context means without involving the step of performing culture/proliferation in vitro.

Mesenchymal stem cells isolated directly from biological tissue containing them may be obtained by a method known per se. For example, the method described in International Patent Publication No. WO2017/094879 may be used to obtain desired cells. Examples of the biological tissue containing mesenchymal stem cells include bone marrow, adipose tissue, blood, placenta, umbilical cord, and dental pulp. Of these, adipose tissue is preferable as a source of the cells because it can be collected by liposuction or lipectomy and there is little risk of causing functional disorder in the living body.

Adipose tissue is a type of biological tissue constituted by fat cells. When used as a source of the above-described cells, there is no particular limitation on the location of the adipose tissue, and the adipose tissue may be, for example, subcutaneous fat, visceral fat, intramuscular fat, or intermuscular fat. Of these, subcutaneous fat is preferable because it can be collected easily under local anesthesia and thus imposes a slight burden on the donor at the time of collecting.

Endometrial epithelial cells can also be prepared by a method known per se (Ye L. et al. (2011), PLoS one, 6:e21136; Jiang X et al. (2021), Bioactive Materials, 6:3935-3946). More specifically, for example, strating from pluripotent stem cells of a human, mouse, or the like, they may be obtained by inducing differentiation of prepared definitive endoderm cells based on the description regarding a known method (D'Amour KA et al. (2005), Nature Biotechnology, 23:1534-1541).

As used herein, the term "organoid" refers to a cell aggregate forming a structure having a new function that is not exhibited by cells constituting the cell aggregate alone. Preferably, the structure is capable of differentiating into an organ when it matures, and whether the structure has such differentiation ability can be determined by, for example, transplanting the structure into a living body and examining whether it can differentiate into an intended organ (if it differentiates into the intended organ, it can be determined that the structure is an organoid).

As used herein, the term "endometrial epithelial organoid" refers to a cell aggregate that expresses at least Epcam, E-cadherin, Cytokeratin, MUC1, an estrogen receptor, and a progesterone receptor and is functionally responsive to estrogen (E2) and progesterone (P4), and in addition, when further stimulated with human chorionic gonadotropin (hCG), placental lactogen (hPL), and a stromal cell (PRL) signal, exhibits characteristics of the gestational endometrium and can synthesize PAEP (glycodelin) and SPP1 (osteopontin). In the artificial uterus according to the present invention, the endometrial epithelial organoid is present in a medium. The medium is not limited to a particular medium as long as the survival of the endometrial epithelial organoid is maintained, and can be selected as appropriate from media to be described below. In one embodiment, the medium may be WNT medium containing 30% KSR (Thermo Fisher), N-acetylcysteine, estrogen, and progesterone (partially modified from the medium described in Bedzhov, 2014, Nature Protocols).

The endometrial epithelial organoid may be produced by a method known per se (e.g., Turco et al., NATURE CELL BIOLOGY 19, 568-577 (2017)). Specifically, for example, first, the uterus is collected from a euthanized mouse, then incised to expose the epithelium, and fragmented in this state. Alternatively, part of human-derived uterine tissue is used. The fragments or tissue is subjected to an enzymatic treatment with Dispase, after which epithelial cells are collected. Next, the epithelial cells are suspended in a basement membrane specimen to be described below (e.g., Matrigel) and cultured in Advanced DMEM/F12 medium (WNT medium) supplemented with WNT3A, R-spondin 1, Noggin, EGF, and Hepes pH 7.4 to produce uterine organoids.

The (endometrial) epithelial cells used in the production of the endometrial epithelial organoid are highly polarized, and compartmentalized into an apical (domain) side facing outward in vivo and a basal (domain) side facing the basement membrane. In an organ (e.g., the uterus) constituted by epithelial cells and the like, the apical (domain) side faces the external environment in vivo whereas the basal (domain) side adheres to the extracellular matrix side of the basement membrane via integrins and their receptors.

The endometrial epithelial organoid of the present invention may have the same polarity as the uterus in vivo (i.e., the apical (domain) side is on the outer side of the organoid facing the external environment and the basal (domain) side is on the inner side of the organoid), or may have the opposite polarity (i.e., the basal (domain) side is on the outer side of the organoid facing the external environment and the apical (domain) side is on the inner side of the organoid).

When it is intended to use the endometrial epithelial organoid of the present invention in screening using fertilized eggs (blastocysts or the like) to be described below, the endometrial epithelial organoid preferably has the same polarity as the uterus in vivo (i.e., the apical (domain) side is on the outer side of the organoid facing the external environment and the basal (domain) side is on the inner side of the organoid). Accordingly, if the polarity of the produced endometrial epithelial organoid is opposite to that of the uterus in vivo (i.e., the basal (domain) side is on the outer side of the organoid facing the external environment and the apical (domain) side is on the inner side of the organoid), the polarity may be controlled so as to be the same as that of the uterus in vivo.

The polarity of the endometrial epithelial organoid can be determined by a method known per se (Forteza R. et al., (2016), Molecular Biology of the Cell, 27:2186-2197). Specifically, for example, when the endometrial epithelial organoid is stained with Hoechst/Phalloidin, the following results are obtained if the endometrial epithelial organoid has the same polarity as that of the uterus in vivo: actin present on the apical (domain) side is stained with Phalloidin and detected on the outer side facing the external environment; and DNA present on the basal (domain) side is stained with the Hoechst dye and detected on the inner side of the organoid. In contrast, if the polarity is opposite to that of the uterus in vivo, DNA present on the basal (domain) side is stained with the Hoechst dye and detected on the outer side facing the external environment, and actin present on the apical (domain) side is stained with Phalloidin and detected on the inner side of the organoid.

The method for controlling the polarity is not limited to a particular method as long as the polarity of the endometrial epithelial organoid can be changed to desired polarity, and in an example, the following method may be used (Co JY. et al., (2019), Cell Reports, 26:2509-2520). Specifically, when it is intended to prepare endometrial epithelial organoids in which the outer side is the apical side, the polarity may be controlled by culturing the organoids in a low attachment or ultra-low attachment culture vessel to be described below. As such a culture vessel, an Ultra-Low Attachment dish (Corning Incorporated), an Ultra-Low Attachment flask (Corning Incorporated), an Ultra-Low Attachment plate (Corning Incorporated), a Prime Surface dish (Sumitomo Bakelite Co., Ltd.), or the like may be used. A medium, culture conditions, and a culture period used for controlling the polarity may be set as appropriate. For example, the medium may be WNT medium (Advanced DMEM/F12 medium supplemented with WNT3A, R-spondin 1, Noggin, EGF, Hepes pH 7.4, and estrogen) to be described below. The culture temperature is, for example, 30°C to 40°C and preferably about 37°C, the culture is performed in an atmosphere of CO₂-containing air in which the CO₂ concentration is preferably about 2% to 5%, and the culture period is one day to several months (e.g., 1, 2, 3, 4, 5, or 6 months) and preferably 2 weeks to 3 months.

In the course of producing the endometrial epithelial organoids of the present invention, uterine tissue from which epithelial cells have been removed is generated. This tissue may be subjected to an enzymatic treatment with collagenase or the like, and endometrial stromal cells as described above may be collected therefrom. The collected stromal cells may be further cultured under adherent conditions (adherent culture) using a culture vessel, medium, and the like to be described below. Specifically, for example, they may be cultured in DMEM medium containing 10% serum in a dish that has been treated so as to promote cell attachment.

In the present specification, the terms "uterine organoid" and "endometrial epithelial organoid" mean the same and can be used interchangeably.

In the artificial uterus of the present invention, an endometrial epithelial organoid produced is arranged so as to be surrounded by a hydrogel mainly composed of extracellular matrix. In the present invention, there is no particular limitation on the state of being "arranged so as to be surrounded by the hydrogel" as long as fertilized egg (blastocyst) implantation into the endometrial epithelial organoid to be described below can occur. In a preferable embodiment, for example, the artificial uterus may be configured such that the hydrogel arranged so as to surround the endometrial epithelial organoid has an opening open to the top thereof, and in a more preferable embodiment, the hydrogel has only one opening. In a still more preferable embodiment, the endometrial epithelial organoid is arranged such that all parts (surfaces) thereof are surrounded by the hydrogel except for one opening.

Examples of the extracellular matrix that can be used in the present invention include laminin (Nat Biotechnol 28, 611-615 (2010)), laminin fragments (Nat Commun 3, 1236 (2012)), basement membrane specimens (Nat Biotechnol 19, 971-974 (2001)), fibronectin, gelatin, collagen, heparan sulfate proteoglycan, entactin, and vitronectin.

The "laminin", which is a heterotrimeric molecule composed of α, β, and γ chains, is extracellular matrix protein with isoforms that differ from each other in the composition of their subunit chains. Specifically, laminin has about fifteen types of isoforms resulting from the combination of various heterotrimer chains, namely, five types of α chains, four types of β chains, and three types of γ chains. Laminin is named according to the number used in each of the α chain (α1 to α5), the β chain (β1 to β4) and the γ chain (γ1 to γ3). For example, laminin composed of the combination of α5 chain, β1 chain, and γ1 chain is named laminin-511 (Nat Biotechnol 28, 611-615 (2010)).

In the present invention, a mammalian laminin is typically used. A mammalian laminin derived from the same species as the cells constituting the endometrial epithelial organoid or as the cells to be cultured may be used. For example, a human laminin (preferably, human laminin-511) is used for culturing human pluripotent stem cells.

The laminin fragments used in the present invention are not particularly limited as long as they allow implantation of a fertilized egg (blastocyst) into the endometrial epithelial organoid described below, but include laminin-111 or an E8 region-containing fragment thereof, laminin-211 or an E8 region-containing fragment thereof (e.g. iMatrix-211), laminin-121 or an E8 region-containing fragment thereof, laminin-221 or an E8 region-containing fragment thereof, laminin-332 or an E8 region-containing fragment thereof, laminin-3A11 or an E8 region-containing fragment thereof, laminin-411 or an E8 region-containing fragment thereof (e.g., iMatrix-411), laminin-421 or an E8 region-containing fragment thereof, laminin-511 or an E8 region-containing fragment thereof (e.g., iMatrix-511, iMatrix-511 silk), laminin-521 or an E8 region-containing fragment thereof, laminin-213 or an E8 region-containing fragment thereof, laminin-423 or an E8 region-containing fragment thereof, laminin-523 or an E8 region-containing fragment thereof, laminin-212/222 or an E8 region-containing fragment thereof, laminin-522 or an E8 region-containing fragment thereof and the like. Among these, laminin-511 or an E8 region-containing fragment thereof is preferred. The E8 fragment of laminin 511 is commercially available and can be purchased, for example, from Nippi, Inc. The laminin or laminin fragment used in the present invention is preferably isolated.

The "basement membrane specimen" in the present invention refers to one that contains basement membrane constituents having functions of controlling epithelial cell-like cell morphology, differentiation, proliferation, motility, functional expression, and the like when desired cells with basement membrane-forming ability are seeded and cultured thereon. The "basement membrane constituents" refer to extracellular matrix molecules in the form of a thin membrane present between the epithelial cell layer and the stromal cell layer or the like in animal tissues. The basement membrane specimen can be produced by, for example, removing cells with basement membrane-forming ability, adhering to supporting tissue via a basement membrane from the supporting tissue using a solution capable of dissolving lipids of the cells or an alkaline solution. Examples of the basement membrane specimen include products commercially available as basement membrane preparations (e.g., Matrigel^{™} (Corning Incorporated, hereinafter also referred to simply as Matrigel)), Geltrex^{™} (Life Technologies), and basement membrane specimens containing extracellular matrix molecules known as basement membrane components (e.g., laminin, type IV collagen, heparan sulfate proteoglycan, entactin, etc.).

Matrigel^{™} is a basement membrane preparation extracted from Engelbreth-Holm-Swam (EHS) mouse sarcoma. Matrigel^{™} contains type IV collagen, laminin, heparan sulfate proteoglycan, and entactin as main components, and in addition to these components, Matrigel^{™} further contains TGF-β, FGF, tissue plasminogen activator, and growth factors naturally produced by EHS tumors. The "growth factor reduced product" of Matrigel^{™} contains the growth factors at lower concentrations than in regular Matrigel^{™}, and the standard concentrations of the growth factors are as follows: less than 0.5 ng/ml for EGF, less than 0.2 ng/ml for NGF, less than 5 pg/ml for PDGF, 5 ng/ml for IGF-1, and 1.7 ng/ml for TGF-β.

Cells (e.g., endometrial stromal cells, (endometrial) epithelial cells, etc.) and organoids (endometrial epithelial organoids) used in the present invention may be cultured in a medium to be described below as needed. The medium may be prepared by adding a medium additive(s) to a basal medium as needed.

Examples of basal media include, but are not limited to, RPMI-1640 medium, Eagle's MEM (EMEM), Dulbecco's modified MEM, Glasgow's MEM (GMEM), α-MEM, medium 199, IMDM, Hybridoma Serum-Free medium, KnockOut^{™} DMEM (KO DMEM), Advanced^{™} medium (e.g., Advanced MEM, Advanced RPMI, Advanced DMEM/F-12), Chemically Defined Hybridoma Serum-Free medium, Ham's Medium F-12, Ham's Medium F-10, Ham's Medium F12K, DMEM/F-12, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's Medium (e.g., Waymouth's MB752/1), CMRL medium (e.g., CMRL-1066), Williams' medium E, Brinster's BMOC-3 Medium, E8 Medium, StemPro 34, MesenPRO RS (these are from Thermo Fisher Scientific), ReproFF2, Primate ES Cell Medium, ReproStem (these are from Reprocell Inc.), ProculAD (Rohto Pharmaceutical Co., Ltd.), MSCBM-CD, MSCGM-CD (these are from Lonza), EX-CELL302 medium (SAFC) or EX-CELL-CD-CHO (SAFC), ReproMed^{™} iPSC Medium (Reprocell Inc.), Cellartis MSC Xeno-Free Culture Medium (Takara Bio Inc.), TESR-E8 (Veritas Corporation), StemFit (registered trademark) AK02N and AK03N (Ajinomoto Co., Inc.), and mixtures thereof.

Furthermore, physiologically active substances, nutritional factors, and the like necessary for survival or proliferation of organoids, cells, etc., can be added to the medium as needed. These medium additives may be added to a medium in advance or may be added during culture of organoids or cells. During culture, a medium additive may be added in any form, such as one solution or a mixed solution of two or more types of solutions, and an additive may be added continuously or intermittently.

Examples of the physiologically active substances include insulin, IGF-1, Wnt (Wnt1, Wnt2, Wnt3, Wnt3a, Wnt7a, etc.), transferrin, albumin, coenzyme Q10, various types of cytokines (interleukins (IL-2, IL-7, IL-15, etc.), stem cell factors (SCFs), activin, etc.), various types of hormones, and various types of growth factors (leukemia inhibitory factors (LIFs), basic fibroblast growth factor (bFGFs), TGF-β, epidermal growth factor (EGF), etc.), Wnt/beta-Catenin signal transduction pathway regulators (R-spondin 1, R-spondin 2, R-spondin 3, etc.), and bone morphogenetic protein (BMP) antagonists (Noggin etc.). Examples of the nutritional factors include sugars, amino acids, vitamins, hydrolysates, and lipids. Examples of the sugars include glucose, mannose, and fructose. One type of sugar may be used, or two or more types of sugars may be used in combination. Examples of the amino acids include L-alanine, L-arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, and L-valine. One type of amino acid may be used, or two or more types of amino acids may be used in combination. The amino acids may be acetylated (N-acetyl-L-cysteine etc.). Examples of the vitamins include d-biotin, D-pantothenic acid, choline, folic acid, myo-inositol, niacinamide, pyridoxal, riboflavin, thiamine, cyanocobalamin, and DL-α-tocopherol. One type of vitamin may be used, or two or more types of vitamins may be used in combination. Examples of the hydrolysates include those obtained by hydrolyzing soybeans, wheat, rice, peas, corn, cottonseeds, and yeast extracts. Examples of the lipids include cholesterol, linoleic acid, and linolenic acid. Examples of polysaccharides include gellan gum, deacylated gellan gum, methylcellulose, carboxymethylcellulose, hydroxyethylcellulose, hydroxypropylcellulose, carboxymethylamylose, xanthan gum, alginic acid, carrageenan, diutan gum, and locust bean gum.

Further, antibiotics such as kanamycin, streptomycin, penicillin, or hygromycin may be added to the medium as needed. When an acidic substance such as sialic acid is added to the medium, it is desired to adjust the pH of the medium to a neutral range suitable for cell growth, i.e., pH 5 to 9, preferably pH 6 to 8.

The medium may be a serum (e.g., fetal bovine serum (FBS), human serum, horse serum)-containing medium or a serum-free medium. FBS is preferable as serum. From the viewpoint of preventing contamination with components derived from different animal species, it is preferable that the medium does not contain serum or serum derived from the same animal species as the cells to be cultured is used. Here, serum-free medium refers to medium that does not contain unadjusted or unpurified serum. The serum-free medium may contain purified blood-derived components or animal tissue-derived components (e.g., growth factors).

The medium used for floating culture may or may not contain serum substitutes, as well as serum. Examples of serum substitutes include albumin, albumin substitutes such as lipid-rich albumin and recombinant albumin, plant starch, dextrans, protein hydrolysates, transferrin, other iron transporters, fatty acids, insulin, collagen precursors, trace elements, 2-mercaptoethanol, 3'-thioglycerol, and equivalents thereof. Specific examples of serum substitutes include serum substitutes prepared using the method described in WO 98/30679, and commercially available knockout Serum Replacement (KSR) (Life Technologies Corporation, Thermo Fisher Scientific Corporation), Chemically-defined Lipid concentrated (Life Technologies Corporation), and L-alanine-L-glutamine dipeptide (e.g., Glutamax (Life Technologies Corporation)), etc. Examples of biologically derived factors include platelet-rich plasma (PRP) and culture supernatant components of human mesenchymal stem cells.

The medium may contain one or more hormones. Examples of hormones include estrogen, progesterone, cortisol, dehydroepiandrosterone, dehydroepiandrosterone sulfate. Further, hormones can be replaced with artificial or natural compounds that have the equivalent steroid structure.

The culture vessel used for culture and the like of cells (e.g., endometrial stromal cells, (endometrial) epithelial cells, etc.) and organoids (endometrial epithelial organoids) used in the present invention is not limited to a particular culture vessel, and may be, for example, a flask, tissue culture flask, dish, petri dish, tissue culture dish, multidish, microplate, microwell plate, micropore, multiplate, multiwell plate, chamber slide, schale, tube, tray, culture bag, or roller bottle.

When the cells are cultured under non-adherent conditions, the culture vessel is preferably a low cell attachment, ultra-low cell attachment, or non-cell attachment culture vessel. The low cell attachment, ultra-low cell attachment, or non-cell attachment culture vessel may be a culture vessel whose surface has been artificially treated so as to exhibit low attachment, ultra-low attachment, or no attachment to cells, or a culture vessel that has not been subjected to an artificial treatment for promoting the attachment to cells (e.g., coating treatment with extracellular matrix or the like). As such a culture vessel, an Ultra-Low Attachment dish (Corning Incorporated), Ultra-Low Attachment flask (Corning Incorporated), Ultra-Low Attachment plate (Corning Incorporated), Prime Surface dish (Sumitomo Bakelite Co., Ltd.), or the like may be used. When cells are cultured under adherent conditions, it is preferable to coat the culture vessel with extracellular matrix (e.g., laminin, collagen, etc.) or the like.

### 2. Method for Producing Artificial Uterus

The present invention provides a method for producing an artificial uterus, including the steps of:
(1) filling a culture vessel with a solution containing endometrial stromal cells and mainly composed of extracellular matrix;
(2) pressing a device including a support with an objective surface and at least one protrusion protruding from the objective surface against the solution filling the culture vessel; and
(3) after the solution filling the culture vessel has turned into a gel, removing the device pressed against the solution.

In one embodiment of the artificial uterus production method of the present invention, the steps (2) and (3) are as follows:
(2') inserting a device including a support with an objective surface and at least one protrusion protruding from the objective surface into the solution filling the culture vessel; and
(3') after the solution filling the culture vessel has turned into a gel, removing the device inserted into the solution.

In the "solution containing endometrial stromal cells and mainly composed of extracellular matrix" used in the present invention, the total amount of the extracellular matrix is not limited to a particular amount as long as the solution can form a hydrogel (can turn into a (hydro)gel). Also, the density (cells/cm³) of the endometrial stromal cells contained in the solution is not limited to a particular density as long as fertilized egg (blastocyst) implantation into the endometrial epithelial organoid to be described below can occur, and may be, for example, in the range of 1 × 10⁶ cells/cm³ to 1 × 10⁷ cells/cm³ and preferably in the range of 2 × 10⁶ cells/cm³ to 3 ×10⁶ cells/cm³.

The device used in the present invention is a device including a support with an objective surface and at least one protrusion protruding from the objective surface.

The support has an objective surface. In the present invention, the "objective surface" is a surface that faces and comes into intimate contact with a target object when forming a hole(s) in the target object. In the present invention, the "target object" is a solution containing endometrial stromal cells and mainly composed of extracellular matrix. At the time of forming a hole(s) in the target object, a gap may be present between the objective surface and the target object. The overall shape of the support is not limited to a particular shape. For example, the support may be in a plate-like shape with a thickness of about 0.5 mm to 1 mm (the shape may also be referred to as a film-like shape, sheet-like shape, or the like depending on the thickness), a columnar shape (cylindrical shape, prism shape, polygonal shape, or the like) that can be easily grasped or held by hand or with an instrument. The outer peripheral shape of the objective surface is not limited to a particular shape as long as it does not interfere with the formation of a hole(s) by the protrusion(s), and may be, for example, circular, oval, or square shape. In one example, the area of the objective surface is approximately 25 to 30 mm².

In the present invention, the "protrusion" has a shape capable of functioning as a die for forming a hole. There is no particular limitation on the length, outer diameter, overall shape, tip shape, and the like of the protrusion as long as fertilized egg (blastocyst) implantation into the endometrial epithelial organoid to be described below can occur in the hole to be formed by the protrusion. The length, outer diameter, overall shape, tip shape, and the like of the protrusion may be modified as appropriate from the intended dimensions of a hole in consideration of shrinkage and the like of the hole after being formed. Also, there is no particular limitation on the arrangement pattern of the protrusions, the center-to-center distance, and the gap between adjacent protrusions as long as fertilized egg (blastocyst) implantation into the endometrial epithelial organoid to be described below can occur in the holes formed using the protrusions. The device used in the present invention has at least one protrusion. A specific structure of the protrusion may be, for example, a cylindrical shape with a length of 1 to 3 mm and an outer diameter of 100 to 200 µm or such a cylindrical shape with its tip being rounded.

In one embodiment of the present invention, the device includes a holding structure that functions as a handle or an engagement portion to be used when holding the support by hand or with an external apparatus. The holding structure may be provided integrally with the support, or may be provided as an independent component connectable to the support. When the support has an overall shape that is easy to hold, the support having such a shape can also be interpreted as being integrated with the holding structure.

The device used in the present invention may be produced, for example, by producing the support, the protrusion, and optionally the holding structure separately and then attaching them together by bonding or the like, or alternatively, by producing some or all of them integrally as a single unit. When it is intended to produce the device as a single unit, the device may be produced using, for example, a 3D printer (inkjet-type, powder modeling-type, powder sintering-type, or the like).

The materials of the support, the protrusion, and the holding structure as the constituent elements of the device used in the present invention are not limited to particular materials as long as a hole in which fertilized egg (blastocyst) implantation into the endometrial epithelial organoid to be described below can occur is formed after performing the steps (2) and (3). Specific examples of the materials include ABS resin (Acrylonitrile-Butadiene-Styrene resin), ASA resin (Acrylonitrile-Styrene-Acrylate resin), PETG resin (polyethylene terephthalate glycol resin), PLA resin (polylactic acid resin), nylon resins, resin materials in which any of these resins and a fiber material such as carbon are used, PP (polypropylene) powder resins, PS (polystyrene) powder resins, and metal materials.

In the step (2) of the present invention, the timing at which the device is pressed against the solution is not limited to a particular timing as long as the device is pressed while at least part of the solution filling the culture vessel remains in the state of solution. Also, in the step (3), the timing at which the device pressed against the solution in the step (2) is removed is not limited to a particular timing as long as fertilized egg (blastocyst) implantation into the endometrial epithelial organoid to be described below can occur in the hole formed after the device is removed.

The above description in the "1. Artificial Uterus" section is incorporated in its entirety in the description regarding the artificial uterus production method according to the present invention.

### 3. Kit for Producing Artificial Uterus

The present invention provides a kit for producing an artificial uterus, including: a device including a support with an objective surface and at least one protrusion protruding from the objective surface; and a solution mainly composed of extracellular matrix. The kit may optionally include endometrial stromal cells, endometrial epithelial organoids, and/or the like.

The above descriptions in the "1. Artificial Uterus" and "2. Method for Producing Artificial Uterus" sections are incorporated in their entirety in the description regarding the artificial uterus production kit according to the present invention.

### 4. Screening Method

The present invention provides a screening method for an agent for treating infertility, including:
(1) bringing a fertilized egg into contact with the artificial uterus of the present invention in the presence or absence of a test substance;
(2) measuring an implantation rate of the fertilized egg in the endometrial epithelial organoid; and
(3) when the implantation rate is higher in the presence of the test substance than in the absence of the test substance in the step (2), selecting the test substance as a candidate substance for a therapeutic or prophylactic medicament for infertility.

In the present invention, the term "fertilized egg" refers to a diploid cell formed through the union of a sperm and an egg and is derived from a mammal. In one embodiment, a fertilized egg is a non-human fertilized egg. In the present invention, the fertilized egg encompasses an early embryo and a blastocyst, and when implantation is intended, it is preferable to use a blastocyst. When a blastocyst is used as a fertilized egg, it is preferable to detach the zona pellucida from the blastocyst. In the present specification, a fertilized egg may also be referred to as an "embryo". In addition to the above, the fertilized egg also encompasses those produced from pluripotent stem cells (Warmflash A., et al., Nature Methods, 11:847-854; Sinunovic M., et al., bioRxiv, 330704; Shao Y., et al., Nature Communications, 8:208; ten Berge D., et al., Cell Stem Cell, 3:508-518; Beccari L., et al., Nature, 562:272-276; van den Brink SC., et al., Development, 144:3894-3906; Harrison SE., et al., Science, 356:eaal1810, Sozen B., et al., Nature Cell Biology, 20:978-989; Rivron NC., et al., Nature, 557:106-111; Kagawa H., et al., Nature, 601:600-605).

The step (1) of the present invention is the step of bringing a fertilized egg into contact with an endometrial epithelial organoid included in the artificial uterus of the present invention. The contact can be achieved by adding the fertilized egg to a medium or the like containing endometrial epithelial organoids. There is no particular limitation on the contact, and the contact period may be, for example, at least 1 day, at least 2 days, at least 3 days, at least 4 days, at least 5 days, at least 6 days, or at least 7 days, and is preferably 1 to 3 days. The temperature at the time of bringing the fertilized egg into contact with the endometrial epithelial organoid is not limited to a particular temperature, and may be about 30°C to 40°C and preferably about 37°C. The culture is performed in an atmosphere of CO₂-containing air in which the CO₂ concentration is preferably about 2% to 5%.

In the screening method according to the present invention, the concentration of the test substance can be adjusted as appropriate according to the type of the compound (solubility, toxicity, etc.).

The measurement of the implantation rate of the fertilized egg in the endometrial epithelial organoid in the step (2) of the present invention can be performed by observing whether the fertilized egg has penetrated into the endometrium of the endometrial epithelial organoid (in the present specification, this may also be referred to as an "implantation-like reaction"). The observation as to whether the implantation-like reaction has occurred may be performed by, for example: 1) observing whether fluorescence emission from the fertilized egg disappears as a result of the penetration using a fluorescence microscope; 2) observing whether fluorescence emission from the fertilized egg has penetrated into the endometrium using a confocal microscope; and 3) observing the occurrence of the phenomenon such as fusing of the embryo and the endometrial epithelial organoid after removing them from the artificial uterus. In the case of observation using fluorescence, it is desirable that the cells on the artificial uterus side and the fertilized egg are stained with different fluorescent dyes. For example, animals adapted to express a fluorescent protein or photoprotein by genetic engineering can be used. For example, fertilized eggs artificially inseminated with sperm collected from Rosa26^{H2B-EGFP/H2B-EGFP} mice express a green fluorescent protein (EGFP), and endometrial epithelial organoids and stromal cells cultured from Rosa26^{mCherry/mCherry} mice express a red fluorescent protein (mCherry). They can also be fluorescently labeled with a fluorescent substance such as Cell Tracker (Thermo Fisher) or Cell Explorer (AAT Bioquest). For example, the observation may be performed by staining the endometrial epithelial organoids and the stromal cells with Cell Explorer^{™} Fixable Live Cell Tracking Kit Green Fluorescence and the fertilized egg with Cell Explorer^{™} Live Cell Tracking Kit Red Fluorescence.

The implantation rate in the absence of the test substance to be used for comparison in the step (3) of the present invention may be measured as a control for each experiment, or may be measured in advance.

Substances obtained by the screening according to the present invention are considered to promote implantation-like reactions also in vivo, and thus are suitable as therapeutic or prophylactic medicaments for infertility or as candidate substances for such medicaments. In the therapeutic or prophylactic medicaments, the substances may serve as a concomitant agent (pharmaceutical) or combined agent (pharmaceutical) to be used in combination with a medicament used in treatments of infertility (e.g., a hormonal agent).

The above descriptions in the "1. Artificial Uterus", "2. Method for Producing Artificial Uterus", and "3. Method for Producing Artificial Uterus" sections are incorporated in their entirety in the description regarding the screening method according to the present invention.

### 4. Agent for Activating Integrins

The present invention provides an agent for activating integrins, containing:
(1) a peptide consisting of KFEEERMRCKWMT;
(2) a peptide consisting of KFEEERSRCKWMT;
(3) a peptide consisting of the amino acid sequence specified in (1) or (2) with deletion, substitution, and/or addition of one to three (i.e., one, two, or three) amino acids; or
(4) the peptide of any one of (1) to (3) with a membrane-permeable molecule bound thereto.

In the present invention, "activating integrins" refers to increasing cell attachment mediated by integrins. The agent for activating integrins according to the present invention increases cell attachment mediated by integrins, thereby promoting the implantation (rate) of a fertilized egg (embryo or blastocyst) into the endometrium. Accordingly, in one embodiment, the agent for activating integrins according to the present invention is an agent for promoting the implantation (rate) of a fertilized egg (embryo or blastocyst) into the endometrium (in the present invention, it may be simply referred to as an "agent for promoting embryo implantation"). Since the agent according to the present invention or the peptide contained in the agent ultimately promotes embryo implantation, fertility treatments and the like can be performed by administering the agent or the peptide to a subject. Thus, in one embodiment, the agent according to the present invention is a therapeutic agent for infertility. Furthermore, the agent according to the present invention may be used in combination with, e.g., hormonal agents used in fertility treatments to be described below, and may be formulated as a concomitant agent or combined agent.

The amino acid sequence specified in (3) above may be, for example: (i) the amino acid sequence of the peptide consisting of KFEEERMRCKWMT (SEQ ID NO: 1) or the peptide consisting of KFEEERSRCKWMT with one to several (two, three, four, or five) amino acids, preferably one to four amino acids, more preferably one to three amino acids, and still more preferably one or two amino acids being substituted with other amino acids and even more preferably with one amino acid being substituted with another amino acid; (ii) the amino acid sequence represented by SEQ ID NO: 1 or 2 with one to several (two, three, four, or five) amino acids, preferably one to four amino acids, more preferably one to three amino acids, still more preferably one or two amino acids, and even more preferably one amino acid being deleted; (iii) the amino acid sequence represented by SEQ ID NO: 1 or 2 with one to several (two, three, four, or five) amino acids, preferably one to four amino acids, more preferably one to three amino acids, still more preferably one or two amino acids, and even more preferably one amino acid being inserted; (iv) the amino acid sequence represented by SEQ ID NO: 1 or 2 with one to several (two, three, four, or five) amino acids, preferably one to four amino acids, more preferably one to three amino acids, still more preferably one or two amino acids, and even more preferably one amino acid being added; or (v) an amino acid sequence resulting from any combination thereof.

Substitution with other amino acids is preferably substitution with amino acids having similar physical and chemical properties ("similar amino acids"), for example, with amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having hydroxy groups (Ser, Thr), and amino acids having a small side chain (Gly, Ala, Ser, Thr, Met). It is predicted that substitution with such similar amino acids does not change the phenotype of proteins (i.e., such substitution is conservative amino acid substitution). Specific examples of conservative amino acid substitution are well known in the art and described in various literatures (see Bowie et al., Science, 247:1306-1310 (1990), for example). When the amino acid sequence has an amino acid(s) substituted, deleted, or inserted as described above, the position(s) of the substituted, deleted, or inserted amino acid(s) is not limited to a particular position(s) as long as such modification can increase cell attachment mediated by integrins. In an embodiment in which an amino acid(s) is inserted, the amino acid sequence specified in (3) above may be the amino acid sequence of SEQ ID NO: 1 or 2 with one or two amino acids being inserted to the N-terminus and/or C-terminus. The amino acids to be substituted may be substituted with unnatural artificial amino acids. In particular, amino acids modified with fluoride (F), chloride (Cl), bromide (Br), or iodide (I) are preferably used for substitution because such amino acids exhibit a marked effect on cell permeability and stability in blood.

The membrane-permeable molecule is not limited to a particular molecule as long as, upon binding to a peptide, it allows the peptide to exhibit increased hydrophobicity and improved affinity for cell membranes. Specific examples of the membrane-permeable molecule include saturated fatty acids, and more specific examples of the same include caprylic acid, capric acid, lauric acid, myristic acid, palmitic acid, and stearic acid. Also, polyethylene glycol may be reversibly bound to the peptide using a method known per se (Utatsu et al., Materials Today Bio, Volume 12, September 2021, 100160).

For example, myristoylation of a peptide, i.e., binding myristic acid to (the N-terminus) of the peptide, and palmitoylation of a peptide, i.e., binding palmitic acid to the peptide, are preferable because they allow the peptide to exhibit increased hydrophobicity and improved affinity for cell membranes.

The agent for activating integrins according to the present invention may further contain a pharmaceutically acceptable carrier and the like to be described below in addition to the peptide of the present invention, and may be administered orally or parenterally to mammals and other targets.

Examples of the dosage form of the agent for activating integrins according to the present invention include those for oral administration, such as tablets (e.g., a sugar-coated tablet, film-coated tablet, sublingual tablet, buccal tablet, and rapid orally disintegrating tablet), pills, granules, powders, capsules (e.g., a soft capsule and microcapsule), syrups, emulsions, suspensions, and films (e.g., an orally disintegrating film and oral mucosa patch film). Examples of the dosage form of the agent for activating integrins according to the present invention further include those for parenteral administration, such as injections, infusions, transdermal agents (e.g., an iontophoresis transdermal agent), suppositories, ointments, nasal agents, transpulmonary agents, eye drops, vaginal suppositories, and vaginal capsules. The agent for activating integrins according to the present invention may also be a controlled-release preparation such as a quick-release preparation or sustained-release preparation (e.g., a sustained-release microcapsule).

The agent for activating integrins according to the present invention can be produced using known production methods commonly used in the field of pharmaceutical formulation (e.g., methods described in the Japanese Pharmacopoeia). When necessary, the agent for activating integrins according to the present invention may contain proper amounts of additives commonly used in the field of pharmaceutical formulation, such as an excipient, binder, disintegrant, lubricant, sweetening agent, surfactant, suspending agent, emulsifying agent, coloring agent, preservative, aromatic agent, taste masking agent, stabilizer, and thickener, as appropriate. Examples of the pharmacologically acceptable carrier include these additives.

For example, a tablet can be produced using additives such as an excipient, binder, disintegrant, and lubricant, and a pill and granules can be produced using additives such as an excipient, binder, and disintegrant. A powder and capsule can be produced using additives such as an excipient, a syrup can be produced using additives such as a sweetening agent, and an emulsion and suspension can be produced using additives such as a suspending agent, surfactant, and emulsifying agent.

Examples of the excipient include lactose, white soft sugar, glucose, starch, sucrose, microcrystalline cellulose, powdered glycyrrhiza, mannitol, sodium hydrogen carbonate, calcium phosphate, calcium sulfate.

Examples of the binder include 5 to 10 wt% starch paste solution, 10 to 20 wt% gum arabic solution or gelatin solution, 1 to 5 wt% tragacanth solution, carboxymethylcellulose solution, sodium alginate solution, and glycerol.

Examples of the disintegrant include starch and calcium carbonate.

Examples of the lubricant include magnesium stearate, stearic acid, calcium stearate, and purified talc.

Examples of the sweetening agent include glucose, fructose, invert sugar, sorbitol, xylitol, glycerol, and simple syrup.

Examples of the surfactant include sodium lauryl sulfate, polysorbate 80, sorbitan mono-fatty acid ester, and polyoxyl stearate 40.

Examples of the suspending agent include gum arabic, sodium alginate, sodium carboxymethylcellulose, methylcellulose, and bentonite.

Examples of the emulsifying agent include gum arabic, tragacanth, gelatin, and polysorbate 80.

For example, when the agent according to the present invention is in the form of a tablet, the tablet can be produced according to a method known per se, for example, by adding, to the peptide of the present invention, an excipient (e.g., lactose, white soft sugar, or starch), disintegrant (e.g., starch or calcium carbonate), binder (e.g., starch, gum arabic, carboxymethylcellulose, polyvinylpyrrolidone, or hydroxypropylcellulose), or lubricant (e.g., talc, magnesium stearate, or polyethylene glycol 6000) and compression molding the resulting mixture, and when necessary, coating the thus-molded mixture by a method known per se for the purpose of taste masking, enteric coating, or sustained release. Examples of the coating agent used for coating include hydroxypropylmethylcellulose, ethylcellulose, hydroxymethylcellulose, hydroxypropylcellulose, polyoxyethyleneglycol, Tween 80, Pluronic F68, cellulose acetate phthalate, hydroxypropylmethylcellulose phthalate, hydroxymethylcellulose acetate succinate, Eudragit (manufactured by Rohm, Germany, methacrylic acid-acrylic acid copolymer) and pigments (e.g., iron oxide red and titanium dioxide).

Examples of the injections include intravenous injections, as well as subcutaneous injections, intradermal injections, intramuscular injections, intraperitoneal injections, and injections for drip infusion.

Such injections are prepared by a method known per se, i.e., by dissolving, suspending, or emulsifying the peptide of the present invention in a sterile aqueous liquid or oily liquid. Examples of the aqueous liquid include physiological saline and isotonic solutions (e.g., D-sorbitol, D-mannitol, and sodium chloride) containing glucose and other auxiliary agents. The aqueous liquid may contain a suitable solubilizing agent such as, for example, alcohol (e.g., ethanol), polyalcohol (e.g., propylene glycol or polyethylene glycol), or a nonionic surfactant (e.g., polysorbate 80 or HCO-50). Examples of the oily liquid include sesame oil and soybean oil. The oily liquid may contain a suitable solubilizing agent. Examples of the solubilizing agent include benzyl benzoate and benzyl alcohol. Such injections may be blended with a buffer (e.g., phosphate buffer or sodium acetate buffer), soothing agent (e.g., benzalkonium chloride or procaine hydrochloride), stabilizer (e.g., human serum albumin or polyethylene glycol), preservative (e.g., benzyl alcohol or phenol), and/or the like. Prepared injection solutions are typically filled into ampoules.

The content of the peptide of the present invention in the agent according to the present invention varies depending on the form of the preparation, but is generally about 0.01 to about 100 wt%, preferably about 2 to about 85 wt%, and more preferably about 5 to about 70 wt% with respect to the whole preparation.

The content of additives in the agent according to the present invention varies depending on the form of the preparation, but is generally about 1 to about 99.9 wt% and preferably about 10 to about 90 wt% with respect to the whole preparation.

The peptide of the present invention is stable and low toxic, and thus can be used safely. The daily dose of the peptide of the present invention varies depending on the condition and body weight of the patient, the type of the compound (amino acids), the administration route, and so forth. For example, when the peptide of the present invention is administered orally to a patient for the purpose of fertility treatment, the daily dose for an adult (body weight: about 60 kg) is about 5 to 500 mg in terms of the peptide of the present invention. The above-described daily dose may be administered as a single dose or may be divided into multiple doses.

When the peptide of the present invention is administered parenterally, it is typically administered in the form of a liquid (e.g., an injection) or vaginal capsule. The single dose of the peptide of the present invention varies depending on the administration target, target organ, symptoms, administration method, and so forth, but typically, it is administered, for example, at a dose of about 0.08 to about 8 mg per kg of body weight through transvaginal administration, transdermal injection, or intravenous injection.

The peptide of the present invention can be used in combination with other drugs. Specifically, the peptide of the present invention can be used in combination with a hormonal agent and the like, for example. In the present specification, other drugs that can be used in combination with the peptide of the present invention may also be referred to as "concomitant drugs".

Examples of the hormonal agent include ovulation inducing agents, hCG preparations, follicular hormone (estrogen) preparations, corpus luteum hormone (progesterone) preparations, and GnRH preparations (GnRH agonists/antagonists). Other examples of the hormonal agent include therapeutic medicaments for hyperprolactinemia (e.g., Cabaser) and therapeutic medicaments for endometriosis and mastopathy (e.g., danazol).

In particular, examples of the follicular hormone (estrogen) preparations include estradiol, estriol, ethinyl estradiol, estradiol cypionate, and estradiol valerate. Examples of the corpus luteum hormone (progesterone) preparations include progesterone, medroxyprogesterone acetate, dydrogesterone, norethisterone, and dienogest.

Using the peptide of the present invention in combination with a concomitant drug brings about the following advantageous effects: (1) as compared with the case where either the peptide of the present invention or the concomitant drug is administered alone, the doses thereof can be reduced; (2) it is possible to select a drug to be used in combination with the peptide of the present invention according to the symptoms of a patient (according to whether the symptoms are mild or severe, for example); (3) it is possible to set a long treatment period; (4) the therapeutic effect can be sustained; and (5) synergistic effects can be obtained by using the peptide of the present invention and the concomitant drug in combination.

Hereinafter, the peptide of the present invention when used in combination with a concomitant drug is referred to as a "concomitant agent according to the present invention". When using the concomitant agent according to the present invention, there is no particular limitation on the administration timing of the peptide of the present invention and the concomitant drug, and they may be administered to an administration target either at the same time or in a staggered manner. In the case of staggered administration, the time difference varies depending on active ingredients to be administered, the dosage forms, and the administration method. For example, when a concomitant drug for improving the condition of the endometrium is to be administered first, the peptide of the present invention may be administered at the following timing after the administration of the concomitant drug: in the case where natural conception is intended, within 1 week to 10 days after the intercourse; and in the case of transferring an artificially inseminated embryo, at the time of transferring the embryo. On the other hand, when the peptide of the present invention is to be administered first, a concomitant drug such as a progesterone preparation for maintaining the pregnancy may be administered after the administration of the peptide of the present invention. The dose of the concomitant drug need only conform to the clinically used dose thereof, and can be selected as appropriate depending on the administration target, administration route, disease, combination, and so forth.

Use of the peptide of the present invention is not limited to use in humans. For example, the peptide of the present invention may also be used to improve the implantation efficiency in livestock such as racehorses and beef cattle.

When the peptide of the present invention is used in combination with a concomitant drug, the administration mode may be as follows, for example: (1) a single preparation obtained by simultaneously formulating the peptide of the present invention and the concomitant drug is administered; (2) two types of preparations obtained by separately formulating the peptide of the present invention and the concomitant drug are administered at the same time through the same administration route; (3) two types of preparations obtained by separately formulating the peptide of the present invention and the concomitant drug are administered in a staggered manner through the same administration route; (4) two types of preparations obtained by separately formulating the peptide of the peptide of the present invention and the concomitant drug are administered at the same time through different administration routes; or (5) two types of preparations obtained by separately formulating the peptide of the present invention and the concomitant drug are administered in a staggered manner through different administration routes (e.g., the peptide of the present invention and the concomitant drug are administered in this order or in reverse order).

The dose of the concomitant drug may be selected as appropriate based on the clinically used dose thereof. The blending ratio of the peptide of the present invention and the concomitant drug can be selected as appropriate according to the administration target, administration route, target disease (in particular, infertility), symptoms, combination, and so forth.

The present invention will be described more specifically with reference to the following examples. It is to be noted, however, that the present invention is not limited to these examples by any means.

### EXAMPLES

### Experiment Animals

All animal experiments were performed at the Animal Experimentation Facility of Kansai Medical University with permission granted by the Institutional Animal Care and Use Committee. Mice of C56BL/6, B6D2F1, Rosa26m^{Cherry/mCherry}, Rosa26^{H2B-EGFP/H2B-EGFP}, and ICR strains were used. The animals used were at 8 to 12 weeks of age.

### Cell Culture

Uteri were collected from euthanized C56BL/6 or Rosa26^{mCherry/mCherry} mice, incised to expose the epithelium, and fragmented in this state. They were then subjected to an enzymatic treatment with Dispase, after which epithelial cells were collected. The epithelial cells were suspended in Matrigel and cultured in Advanced DMEM/F12 medium (WNT medium) supplemented with WNT3A, R-spondin 1, Noggin, EGF, and Hepes pH 7.4 to produce uterine organoids (Turco, 2017, Nature Cell Biology). Uterine tissues from which the epithelial cells had been removed were subjected to an enzymatic treatment with collagenase, after which stromal cells were collected. The stromal cells were cultured in DMEM medium containing 10% serum in dishes having been treated so as to promote cell attachment. The uterine organoids and stromal cells derived from the C56BL/6 mice were fluorescently labeled with Cell Explorer^{™} Live Cell Tracking Kit Green Fluorescence (AAT Bioquest) immediately before performing an artificial implantation experiment.

### Embryo Culture

B6D2F1 female mice were administered CARD HyperOva (Kyudo Co., Ltd.), and further administered human chorionic gonadotropin (ASKA Pharmaceutical Co., Ltd.) 48 hours later. Thereafter, they were mated with B6D2F1 male mice. The day after next, the female mice were euthanized, and two-cell stage embryos were collected from their oviducts. The embryos were cultured in KSOM medium (ARK Resource Co., Ltd.) for 3 days to allow them to develop into blastocysts, which were fluorescently labeled with Cell Explorer^{™} Live Cell Tracking Kit Red Fluorescence (AAT Bioquest) immediately before performing an artificial implantation experiment. For artificial insemination, Rosa26^{H2B-EGFP/H2B-EGFP} male mice at 8 weeks of age or older were euthanized, and sperm was collected from their cauda epididymides and cryopreserved using CARD FERTIUP Mouse Sperm Cryopreservation Solution (Kyudo Co., Ltd.). Subsequently, B6D2F1 female mice were administered CARD HyperOva (Kyudo Co., Ltd.), and further administered human chorionic gonadotropin (ASKA Pharmaceutical Co., Ltd.) 48 hours later. On the next day, the female mice were euthanized, and unfertilized eggs were collected from their oviducts. The unfertilized eggs were fertilized with the sperm that had been thawed and then recovered in CARD FERTIUP mouse sperm pre-culture medium, whereby fertilized eggs fluorescently labeled with EGFP were obtained. The fertilized eggs were allowed to develop into blastocysts.

### Example 1: Mini-Uterus and In Vitro Implantation Experiment

The uterine organoids were spherical cell masses composed of a single columnar epithelial cell layer and present in Matrigel (FIG. 1A). The uterine organoids together with the Matrigel were suspended in PBS containing 5 mM EDTA and rotated for 60 minutes at 4°C to dissolve the Matrigel. The uterine organoids were added to a 96-well ultra-low attachment dish (costar) in such a manner that each well contained one uterine organoid, and cultured in WNT medium containing estrogen for 4 days to reverse the polarity. The polarity of epithelial cells can be determined from the positional relationship between the nuclei stained with Hoechst and actin filaments stained with Phalloidin. In the uterine organoids cultured in the Matrigel, nuclei and actin filaments were oriented from the outside toward the inside of the sphere (on the right in FIG. 1F), whereas, in the uterine organoids cultured in the ultra-low attachment dish, nuclei and actin filaments were oriented from the inside toward the outside of the sphere (on the left in FIG. 1F). The actin filament side is the luminal side, which is the side to be in contact with embryos. Thereafter, the uterine organoids were further cultured in WNT medium containing estrogen and progesterone for 1 day. A structure with holes having a diameter of 200 µm was produced by suspending stromal cells in Matrigel, placing the resulting suspension in a glass-bottom dish, and further, solidifying the suspension with a die produced using a 3D printer placed thereon (FIGs. 1C to 1E). The uterine organoids and the blastocysts from which the zona pellucida had been detached were placed in the holes (FIGs. 1B, 2, and 3) and cultured using WNT medium containing 30% KSR (Thermo Fisher), N-Acetylcysteine, estrogen, and progesterone (Bedzhov, 2014, Nature Protocols, partially modified, implantation medium). A confocal fluorescence microscope FV3000 (Olympus Corporation) was used to observe implantation-like reactions.

The confocal fluorescence microscope FV3000 was equipped with a simple CO₂ incubator (Tokai Hit Co., Ltd.), thereby allowing observation of implantation-like reactions to be performed in an environment similar to that in vivo, namely, in 5% CO₂ at 37°C. Three-dimensional fluorescence observation was performed for 3 days using a 488 nm laser for EGFP and Cell Explorer^{™} Live Cell Tracking Kit Green Fluorescence and using a 561 nm laser for mCherry and Cell Explorer^{™} Live Cell Tracking Kit Red Fluorescence. The blastocysts were initially in contact with the uterine organoids and gradually penetrated into the uterine organoids (FIG. 4A). Interactions with the surrounding stromal cells were also observed (FIG. 4A). In addition, mCherry fluorescence was observed in the blastocysts. This suggests that epithelial cells are removed by phagocytosis to allow penetration (Fig. 4B). Also, the above observation results were also observed in the blastocysts and the uterine organoids/stromal cells stained with Cell Explorer^{™} Live Cell Tracking Kit Green Fluorescence and Red Fluorescence (FIG. 5).

### Example 2: Verification of In Vitro Implantation Experiment

When no implantation-like reaction occurred in the in vitro implantation experiment, the embryo (the left panel in FIG. 6A) and the uterine organoid (the right panel in FIG. 6A) removed from the experimental system remain separated from each other. In contrast, when an implantation-like reaction occurred, the embryo removed from the experimental system was fused with the uterine organoid so as not to separate from each other (FIG. 6B). Seventy-two hours after placing the blastocysts in the in vitro implantation experimental system, all the cells were removed from the system and treated with trypsin to disassociate them into single cells in order to perform single-cell RNA expression analysis. Of the thus-obtained single cells, dead cells were fluorescently stained with 7-AAD, and living cells were separated and collected using a cell sorter (SH800S, Sony Corp.). Each one of the collected cells was tagged with RNA using Chromium (10x Genomics) at the Genome Information Research Center, Research Institute for Microbial Diseases, Osaka University, and next-generation sequencing was performed. Gene expression data was analyzed using SEURAT (https://satijalab.org/seurat/). Based on the gene expression patterns, the analyzed cell groups could be classified into three types, namely, placenta-like cells, endometrial stromal cells, and endometrial epithelial cells (FIG. 6C). These three types of cells were considered to be derived from the blastocysts, the stromal cells, and the uterine organoids added to the in vitro implantation experiment system, respectively. In addition to these cells, cells expressing the following genes were scattered: genes involved in stem cell maintenance, such as Prdm16, Klf4, and Rexo1; a gene involved in endoderm formation, such as Gata6; a gene involved in gastrulation, such as Nodal; and genes involved in placentation, such as Plac8, Klf4, and Sdc1. This suggests that embryo development had progressed beyond the blastocyst stage (FIG. 4D).

### Example 3: Expression of Implantation-related Genes

Through the single-cell RNA expression analysis of Example 2, the expression of genes that are known to be involved in implantation was confirmed. Expression of WNT signals (FIG. 7A), Notch signals (FIG. 7B), metalloproteases (FIG. 7C), TGFβ (FIG. 7D), LIF (FIG. 7E), an integrin (FIG. 7F), and the like was observed in both the embryo-derived cells and the endometrium-derived cells, and this suggests that the phenomena observed in Examples 1 and 2 were reactions equivalent to implantation occurring in vivo.

### Example 4: Peptide Activity Measurement (Overview)

The activity of peptides was detected by measuring their ability to form integrin activation complexes using AlphaScreen. Out of over 60,000 types of peptides, Iznm-1 (Myr-KFEEERMRCKWMT (MW: 1984.45)) and Iznm-2 (Myr-KFEEERSRCKWMT (MW: 1940.33)), which exhibited the highest integrin activation ability, were found. The measurement confirmed that Iznm-1 can exhibit approximately 5-fold activation effect and Iznm-2 can exhibit approximately 14-fold activation effect at a concentration of 8 µM (FIG. 8).

### Example 5 (1): Increase in Activated Integrin β1 by Peptide Medicament

In order to examine whether the peptide medicament Iznm-2 actually causes integrin activation in embryos, blastocysts from which the zona pellucida had been removed were added to glass-bottom dishes (Matsunami) and cultured in implantation medium for 3 days. The liquid culture medium was supplemented with an antibody (MAB2259Z, Merck) recognizing activated integrin β1 and an Alexa594-conjugated anti-mouse secondary antibody. The experiment was performed with respect to an experimental group with the addition of Iznm-2 and a control group without the addition of Iznm-2. After the culture, the embryos were immobilized with 4% formaldehyde, and the nuclei were stained with Hoechst. FV3000 or a fluorescent microscope CKX-53 (Olympus Corporation) was used for observation. As a result, cells more strongly fluorescently stained as compared to cells in the control group (FIGs. 8A and 8C) were detected in the experimental group (FIGs. 8B and 8D).

### Example 5 (2): Promotion of Attachment of Embryos to Glass Surface by Peptide Medicament

In the experiment in (1) of Example 5, the embryos in the control group did not attach to the glass surface, whereas the embryos in the experimental group with the addition of Iznm-2 attached to the glass surface (FIGs. 10A and 10B). The rate of attachment was 0/13 in the control group and 13/14 in the Iznm-2-added group, and a significant difference was detected between the two groups (p < 0.01, chi-square test, FIG. 10C).

### Example 6: Effect of Peptide Medicament in In Vitro Implantation System

In the in vitro implantation system of the present invention, the success or failure of an implantation-like reaction was determined based on the success or failure of fusion between an embryo and a uterine organoid as shown in FIGs. 6A and 6B (FIG. 11). In the control group, an implantation-like reaction was observed at a rate of 5/22 (22.7%). In contrast, in an experiment system adapted to exclude stromal cells from the in vitro implantation system, an implantation-like reaction was observed at a rate of 1/22 (4.5%) (p < 0.01, chi-square test and residual analysis). In an Iznm-1-added experiment, the rate was 6/14 (42.9%), and in an Iznm-2-added experiment, the rate was 12/19 (63.2%), and a significant difference was detected in the latter (p < 0.01, chi-square test and residual analysis).

### Example 7: Effect of Peptide Medicament on Embryo Development

In order to examine the effect of the peptide medicament on embryo development, the following experiment was performed. ICR male mice at 8 weeks of age were vasectomized. ICR female mice in proestrus were mated with the vasectomized male mice, and the female mice having developed a copulation plug on the following day were used as pseudopregnant female mice. Two days after confirming the presence of the copulation plug, B6D2F 1 mouse embryos that had developed into blastocysts were transferred to the female mice. The blastocysts were added to KSOM medium supplemented with 32 µM Iznm-2 immediately before the transfer, and the embryos were transferred together with the medium. The amount of the medium injected into the uterus at the time of transfer was 5 µl or less. The embryo transfer was performed in the following manner. The pseudopregnant female mice were anesthetized, and the skin on one side in the dorsum and the abdominal wall of each mouse were incised to expose the uterus to the outside of the body. The uterus was pierced through with a 26-G needle, and the embryos were injected into the thus-formed hole together with the medium using a glass tube. After the transfer, the uterus was returned to the body, and the incision was sutured. The transferred embryos were allowed to develop in the recipient mice until E14. Thereafter, the surrogate mice were euthanized, and the embryos were removed from the bodies and their development was examined. The removed embryos were found to have developed normally, and this suggests that Iznm-2 does not greatly affect embryo development (FIG. 14).

### Example 8: Effect of Peptide Medicament on Embryo Transfer

In order to examine the effect of the peptide medicament on embryo transfer, the following experiment was performed. ICR male mice at 8 weeks of age were vasectomized. ICR female mice in proestrus were mated with the vasectomized male mice, and the female mice having developed a copulation plug on the following day were used as pseudopregnant female mice. Two days after confirming the presence of the copulation plug, B6D2F 1 mouse embryos that had developed into blastocysts were transferred to the female mice. The blastocysts were added to KSOM medium with 8 µM Iznm-2 or without 8 µM Iznm-2 (control) immediately before the transfer, and the embryos were transferred together with the medium. The amount of the medium injected into the uterus at the time of transfer was 5 µl or less. The blastocysts at 4.5 days post-coitum were transferred to the pseudopregnant mice at 2.5 days post-coitum. With such a difference in the number of days post-coitum, an embryo transfer experiment was performed before the uterus was fully prepared for implantation, thereby reproducing a mismatch between the timing at which the endometrium is ready to accept a fertilized egg and the timing when the fertilized egg reaches the endometrium (the so-called "implantation window shift"), which is one of the causes of infertility (FIG. 12). The embryo transfer was performed in the following manner. The pseudopregnant female mice were anesthetized, and the skin on one side in the dorsum and the abdominal wall of each mouse were incised to expose the uterus to the outside of the body. The uterus was pierced through with a 26-G needle, and the embryos were injected into the thus-formed hole together with the medium using a glass tube. After the transfer, the uterus was returned to the body, and the incision was sutured. The transferred embryos were allowed to develop in the recipient mice until E15.5. Thereafter, the surrogate mice were euthanized, and the embryos were removed from the bodies and the implantation rate and their development were examined (FIGs. 13A and 13B). As a result, it was found that, in the control group, none of the 24 transferred embryos had successfully implanted, whereas, in the Iznm-2-added group, 22 out of the 41 transferred embryos had successfully implanted and 12 out of them had developed normally (FIG. 13C). The implanted embryos were divided into two groups: those that did not develop at all and those that developed correctly (FIG. 13D). It is speculated that these results are consistent with the all-or-none principle, which refers to the concept that effects of drugs during the early stage of human pregnancy are divided into two: not at all affecting the embryo development or totally preventing the embryo development.

### Example 9: Effect of Peptide Medicament on Embryo Development

The development of the E15.5 embryos obtained in Example 8 was examined. The embryos exhibited normal morphology (FIG. 14A). Paraffin blocks of the embryos were prepared, and the sections of the embryos were stained with hematoxylin and eosin. As a result, normal development of organs such as the brain, heart, and digestive organs was confirmed (FIG. 14B).

### Example 10: Effects of Peptide Medicament on Maternal Body

An excess amount of Iznm-2 was administered intraperitoneally to female mice to examine the side effects of the peptide medicament. In Example 8, 8 µM Iznm-2 was administered together with 5 µl or less of the embryos to the uterus of each mouse. In the present experiment, Iznm-2 was administered in such a manner that the concentration thereof would be 8 µM with respect to the total weight of each mouse. Iznm-2 was dissolved in corn oil. As a control, corn oil without the peptide medicament was administered. Blood samples were collected 24 hours after the administration, and blood components serving as indicators of the condition of the liver and kidney were analyzed.

Table 1 shows the results of blood tests performed on the mice (maternal bodies) intraperitoneally administered the peptide medicament of the present invention (Iznm-2), in comparison with the control (the mice administered the corn oil).

**[Table 1]**

| | Liver & Kidney | Liver | Liver | Kidney | Liver | Liver | Liver & Kidney | Liver | Liver | Liver | Liver | Liver |
|---|---|---|---|---|---|---|---|---|---|---|---|---|
| | TP (g/dL) | ALB (g/dL) | A/G | CRE (mg/dL) | ALT (IU/L) | ALP (IU/L) | LAP (IU/L) | γ-GT (IU/L) | ChE (IU/L) | T-BIL (mg/dL) | D-BIL (mg/dL) | I-BIL (mg/dL) |
| Corn oil | 4.85 | 3.25 | 2.05 | 0.115 | 45 | 419.5 | 24 | 3 | 29.5 | 0.08 | 0.02 | 0 |
| 8 µM Iznm-2 | 5.1 | 3.3 | 1.85 | 0.12 | 38 | 463 | 26 | 3 | 32.5 | 0.08 | 0.02 | 0.02 |

Data shown in Table 1 confirms that the peptide medicament of the present invention has no notable adverse effects on maternal bodies.

It can be seen from the results of the above examples that the peptide of the present invention is extremely safe for maternal bodies and embryo development. It also can be seen that the peptide of the present invention can directly increase the implantation rate by improving the attachment of the embryo to the uterus, even if the uterus is not fully prepared for implantation.

### INDUSTRIAL APPLICABILITY

The present invention is useful because it can improve the embryo implantation rate after natural conception, artificial insemination, and the like by activating integrins. In addition, since the agent according to the present invention can be used in combination with medicaments used in conventional fertility treatments, it can serve as one of solutions to the declining birthrate and also contributes to reduction of cost for fertility treatments, as well as reduction of increasing subsidies and the like for such treatments. Moreover, the present invention enables quick and reliable evaluation of substances that can improve the embryo implantation rate and thus is very useful for obtaining such substances.

The present application claims priority based on Japanese Patent Application No. 2022-183639 filed in Japan (filing date: November 16, 2022), the disclosure of which is incorporated herein in its entirety by reference.

## Claims

1. An agent for promoting embryo implantation or treating infertility, comprising:
(1) a peptide consisting of KFEEERMRCKWMT;
(2) a peptide consisting of KFEEERSRCKWMT;
(3) a peptide consisting of the amino acid sequence specified in (1) or (2) with deletion, substitution, and/or addition of one to three amino acids; or
(4) the peptide of any one of (1) to (3) with a membrane-permeable molecule bound thereto.

2. The agent according to claim 1, wherein the peptide with the membrane-permeable molecule bound thereto is a myristoylated peptide.

3. The agent according to claim 1 or 2, further comprising a hormonal agent.

4. An agent for activating integrins, comprising:
(1) a peptide consisting of KFEEERMRCKWMT;
(2) a peptide consisting of KFEEERSRCKWMT;
(3) a peptide consisting of the amino acid sequence specified in (1) or (2) with deletion, substitution, and/or addition of one to three amino acids; or
(4) the peptide of any one of (1) to (3) with a membrane-permeable molecule bound thereto.

5. An artificial uterus comprising:
a hydrogel containing endometrial stromal cells and mainly composed of extracellular matrix; and
an endometrial epithelial organoid arranged so as to be surrounded by the hydrogel,
wherein the hydrogel arranged so as to surround the endometrial epithelial organoid has an opening open to the top thereof.

6. The artificial uterus according to claim 5, wherein the extracellular matrix contains laminin and/or a fragment thereof.

7. The artificial uterus according to claim 5, wherein an outer side of the endometrial epithelial organoid is an apical side.

8. A kit for producing an artificial uterus, comprising:
a device including a support with an objective surface and at least one protrusion protruding from the objective surface; and
a solution mainly composed of extracellular matrix.

9. A method for producing an artificial uterus, comprising the steps of:
(1) filling a culture vessel with a solution containing endometrial stromal cells and mainly composed of extracellular matrix;
(2) pressing a device including a support with an objective surface and at least one protrusion protruding from the objective surface against the solution filling the culture vessel; and
(3) after the solution filling the culture vessel has turned into a gel, removing the device pressed against the solution.

10. A screening method for an agent for treating infertility, comprising the steps of:
(1) bringing a fertilized egg into contact with the artificial uterus according to claim 5 in the presence or absence of a test substance;
(2) measuring an implantation rate of the fertilized egg in the endometrial epithelial organoid; and
(3) when the implantation rate is higher in the presence of the test substance than in the absence of the test substance in the step (2), selecting the test substance as a candidate substance for a therapeutic or prophylactic medicament for infertility.
